# EUROPEAN PATENT APPLICATION

(11) **EP 4 130 877 A1**
(43) Date of publication of application: **08.02.2023**
(21) Application number: 21779038.5
(22) Date of filing: 11.03.2021
(51) Int. Cl.: G03F 7/004, G03F 7/027, G02B 5/20, C09B 11/28

(54) **NEGATIVE RESIST COMPOSITION**

(30) Priority: 31.03.2020 JP 2020064631
(71) Applicant: SUMITOMO CHEMICAL COMPANY, LIMITED, Tokyo 103-6020 (JP)
(72) Inventor: NAKAYAMA, Tomohiro, Osaka-shi, Osaka 554-8558 (JP); AKASAKA, Tetsuo, Osaka-shi, Osaka 554-8558 (JP); AOKI, Takuma, Osaka-shi, Osaka 554-8558 (JP); MIYAZAWA, Taku, Osaka-shi, Osaka 554-8558 (JP)
(74) Representative: Vossius & Partner Patentanwälte Rechtsanwälte mbB
(86) International application number: PCT/JP2021/009752
(87) International publication number: WO 2021/200009

(57) **Abstract**

It is an object of the present invention to provide a negative resist composition that is useful for producing a color filter with a high transmittance of light at a wavelength of 440 nm. A negative resist composition of the present invention comprises a colorant and a resin, wherein the colorant contains a xanthene dye, and a cured film formed from the negative resist composition exhibits a spectrum that satisfies the following condition 1:
[condition 1]
a maximum absorption wavelength is present in a wavelength range of 500 to 580 nm, and based on a transmittance at the maximum absorption wavelength being 5%, a transmittance at a wavelength of 440 nm is 80% or more and a transmittance at a wavelength of 620 nm is 80% or more.

## Description

### Technical Field

The present invention relates to a negative resist composition .

### Background Art

Coloring compositions are used for producing color filters used in liquid crystal display device and the like. As such coloring compositions, coloring compositions containing quinacridone pigments are known (Patent Literature 1).

### Citation List

### Patent Literature

Patent Literature 1: Japanese Patent Laid-Open No. 2019-109487

### Summary of Invention

### Technical Problem

It has been known that magenta color can be obtained by mixing blue and red colors. However, in conventional magenta color filters, the transmittance of light at a wavelength of 440 nm is insufficient. It is an object of the present Invention to provide a negative resist composition that is useful for producing a color filter with a high transmittance of light at a wavelength of 440 nm.

### Solution to Problem

The present invention includes the following inventions :
[1] A negative resist composition comprising: a colorant and a resin,
   wherein the colorant contains a xanthene dye, and
   a cured film formed from the negative resist composition exhibits a spectrum that satisfies the following condition 1:
      [condition 1]
      a maximum absorption wavelength is present in a wavelength ränge of 500 to 580 nm, and based on a transmittance at the maximum absorption wavelength being 5%, a transmittance at a wavelength of 440 nm is 80% or more and a transmittance at a wavelength of 620 nm is 80% or more.
[2] The negative resist composition according to [1], wherein the transmittance at a wavelength of 620 nm is 90% or more.
[3] The negative resist composition according to [1] or [2], wherein the xanthene dye is represented by formula (I): 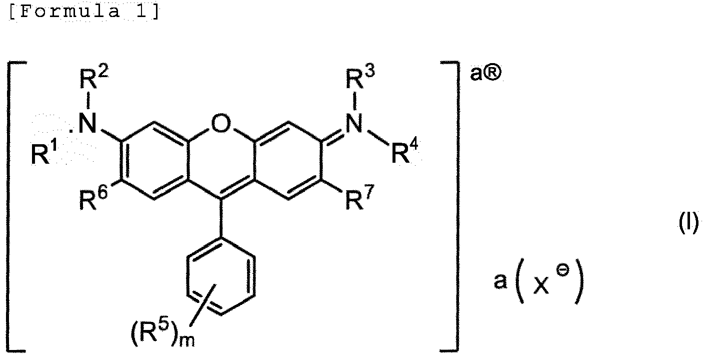
   wherein R¹ to R⁴ each independently represent a hydrogen atom, a monovalent saturated hydrocarbon group having 1 to 20 carbon atoms and optionally having a Substituent, or a monovalent aromatic hydrocarbon group having 6 to 20 carbon atoms and optionally having a substituent, and -CH ₂- contained in the saturated hydrocarbon group is optionally replaced with -0-, -CO-, or -NR¹¹-;
   R⁵ represents -OH, -SO ₃", -SO₃H, -SO₃-Z⁺, -CO₂H, -C0 ₂~ Z⁺, -CO₂R⁸, -SO₃R⁸, or -SO₂NR⁹R¹⁰;
   R⁶ and R⁷ each independently represent a hydrogen atom or an alkyl group having 1 to 6 carbon atoms;
   m represents an integer of 0 to 5; when m is 2 or more, a plurality of R⁵ may be the same or different;
   a represents an integer of 0 or 1;
   X represents a halogen atom;
   Z⁺ represents ⁺N(R¹¹)_{4f} Na⁺, or K⁺, and four R¹¹ may be the same or different;
   R⁸ represents a monovalent saturated hydrocarbon group having 1 to 20 carbon atoms, and a hydrogen atom contained in the saturated hydrocarbon group is optionally replaced with a halogen atom;
   R⁹ and R¹⁰ each independently represent a hydrogen atom or a monovalent saturated hydrocarbon group having 1 to 20 carbon atoms and optionally having a Substituent, and -CH₂- contained in the saturated hydrocarbon group is optionally replaced with -0-, -CO-, -NH-, or -NR⁸-; and R⁹ and R¹⁰ are optionally bonded to each other to form a 3- to 10-membered heterocycle together with the adjacent nitrogen atom; and
   R¹¹ represents a hydrogen atom, a monovalent saturated hydrocarbon group having 1 to 20 carbon atoms, or an aralkyl group having 7 to 10 carbon atoms.
[4] The negative resist composition according to [3], wherein the xanthene dye is represented by formula (la) and/or formula (Ibl): 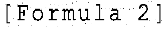 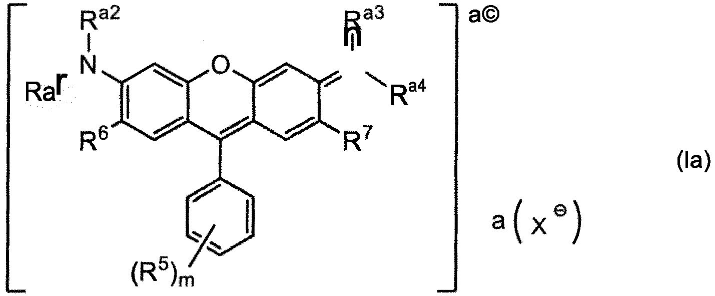 wherein :
   R^{al} and R^{a4} are each independently a monovalent aromatic hydrocarbon group optionally having two or less monovalent saturated aliphatic hydrocarbon groups having 1 to 4 carbon atoms;
   R^{a2} and R^{a3} are each independently a hydrogen atom, a methyl group, or an ethyl group; and
   R⁵ to R⁷, m, a, and X represent the same meanings as the above; 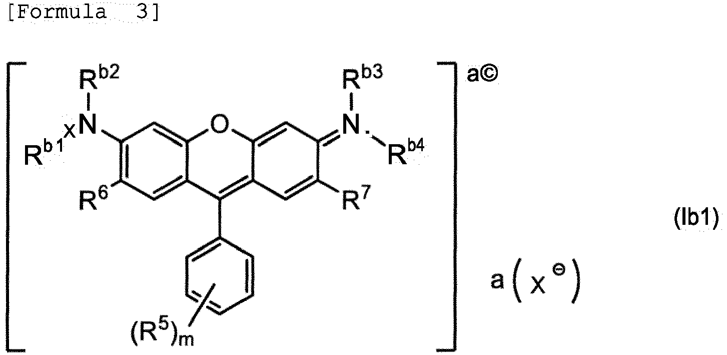
   wherein R^{bl} to R^{b4} each independently represent a hydrogen atom, a monovalent saturated hydrocarbon group having 1 to 20 carbon atoms and optionally having a Substituent, or a monovalent aromatic hydrocarbon group having 6 to 20 carbon atoms and optionally having a substituent;
   at least one saturated hydrocarbon group or aromatic hydrocarbon group contained in R^{bl} to R^{b4} has a halogen atom, -OH, -OR⁸, -CO₂H, -CO₂R⁸, -SO₃", -SO₃H, -SO₃~Z⁺, -SR⁸, -SO₂R⁸, -SO₃R⁸, -SO₂NR⁹R¹⁰, or -Si (OR¹²) (OR¹³) (OR¹⁴) as a substituent, or at least one aromatic hydrocarbon group contained in R^{bl} to R^{b4} has three or more monovalent saturated aliphatic hydrocarbon groups having 1 to 4 carbon atoms as substituents;
   R¹², R¹³, and R¹⁴ each independently represent a monovalent saturated hydrocarbon group having 1 to 4 carbon atoms, and a hydrogen atom contained in the saturated hydrocarbon group is optionally replaced with a halogen atom; and
   R⁵ to R¹⁰, m, a, and X represent the same meanings as the above .
[5] The negative resist composition according to [4], wherein the xanthene dye is a combination of two or more selected from compounds represented by formula (Ibl), or a combination of a compound represented by formula (Ibl) and a compound represented by formula (la) .
[6] The negative resist composition according to any one of [1] to [5], wherein the colorant is a dye alone or a combination of a dye and a red pigment or a purple pigment.
[7] The negative resist composition according to any one of [1] to [6], further comprising a polymerizable compound and a polymerization Initiator.
[8] The negative resist composition according to any one of [1] to [7], wherein the negative resist composition is capable of forming a cured film with a film thickness of 1.5 pm or less.
[9] A color filter formed from the negative resist composition according to any one of [1] to [8].
[10] A display device comprising the color filter according to [9] .

### Advantageous Effects of Invention

A color filter with a high transmittance of light at a wavelength of 440 nm is obtained from the negative resist composition of the present invention.

### Description of Embodiments

### <Negative Resist Composition>

The negative resist composition of the present invention is a negative resist composition comprising a colorant (A) and a resin (C), wherein the colorant (A) contains a xanthene dye, and a cured film formed from the negative resist composition exhibits a spectrum that satisfies the following condition 1:
[condition 1]
a maximum absorption wavelength is present in a wavelength ränge of 500 to 580 nm, and based on a transmittance at the maximum absorption wavelength being 5%, a transmittance at a wavelength of 440 nm is 80% or more and a transmittance at a wavelength of 620 nm is 80% or more.

Because of the above features, a magenta color filter with a high transmittance of light at a wavelength of 440 nm is obtained. The negative resist composition means a resist having the property that the solubility of an exposed area in a developing solution decreases, so that the exposed area remains after development. The negative resist composition is preferred over a positive resist composition in which the solubility of an exposed area in a developing solution is higher, because the negative resist composition tends to provide a broader ränge of process conditions from exposure to development for allowing a pattern to be finished into a desired dimensional ränge.

The spectrum of the cured film can be obtained by preparing the cured film on a glass Substrate under the following conditions, and measuring the spectrum with a colorimeter. The Colorimeter may be OSP-SP-200 (manufactured by Olympus Corporation), for example.

### [Preparation Method of Cured Film]

The negative resist composition is applied onto a glass Substrate having a 5-cm square size by a spin coating method, and then subjected to pre-baking at 100°C for 3 minutes to form a coloring composition layer. After being left to cool, the coloring composition layer is subjected to light Irradiation in an exposure amount of 60 mJ/cm² (basis: 365 nm) under an air atmosphere using an exposure device. Then, the coloring composition layer is subjected to post-baking at 230°C for 20 minutes.

The transmittances of the cured film may be obtained by adjusting the film thickness of the cured film so that the transmittance at the maximum absorption wavelength is 5%, and then measuring the transmittance at a wavelength of 440 nm and the transmittance at a wavelength of 620 nm. Alternatively, the transmittances of the cured film may be obtained without adjusting the film thickness of the cured film as described above, by measuring the transmittance at the maximum absorption wavelength, the transmittance at a wavelength of 440 nm, and the transmittance at a wavelength of 620 nm, and then calculating the transmittance at a wavelength of 440 nm and the transmittance at a wavelength of 620 nm so that the transmittance at the maximum absorption wavelength is 5%.

The transmittance at a wavelength of 440 nm is 80% or more, preferably 85% or more, more preferably 90% or more, and still more preferably 92% or more. The upper limit is not particularly limited, and may be 100% or less, or may be 99% or less.

The transmittance at a wavelength of 620 nm is 80% or more, preferably 90% or more, more preferably 93% or more, still more preferably 95% or more, and particularly preferably 97% or more. The upper limit is not particularly limited, and may be 100% or less.

Hereinafter, each of the components will be described in detail. Herein, compounds mentioned as examples of each component can be used singly or in combinations of a plurality of kinds, unless otherwise specified.

### <Colorant A>

The colorant (A) contains a xanthene dye (Al). The content of the colorant (A) is preferably 5 to 60% by mass, more preferably 8 to 55% by mass, and still more preferably 10 to 50% by mass, with respect to a solid content of the negative resist composition. When the content of the colorant falls within the above-mentioned ränge, the color density as a color filter can be improved, and a required amount of the resin and the like can be incorporated into the composition. As used herein, the solid content of the negative resist composition means the total amount of components obtained by removing the solvent from the negative resist composition of the present invention. The solid content and the content of each of the components with respect thereto can be measured by known analysis means such as liquid chromatography or gas chromatography.

### <Xanthene Dye (Al)>

The xanthene dye (Al) is a dye containing a compound having a xanthene skeleton in its molecule. The xanthene dye (Al) is preferably a dye containing a compound represented by formula (I) (hereinafter, sometimes referred to as "compound (I))". 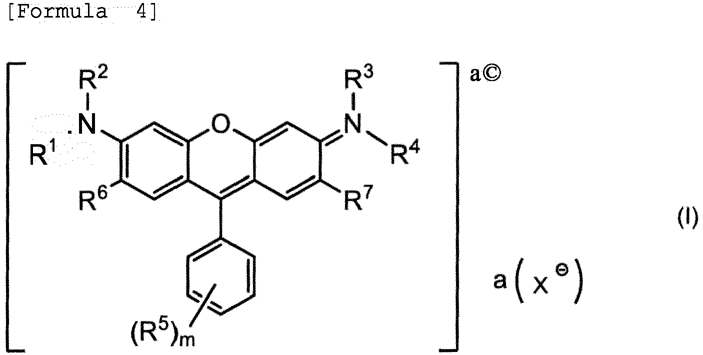
wherein R¹ to R⁴ each independently represent a hydrogen atom, a monovalent saturated hydrocarbon group having 1 to 20 carbon atoms and optionally having a Substituent, or a monovalent aromatic hydrocarbon group having 6 to 20 carbon atoms and optionally having a Substituent, and -CH₂- contained in the saturated hydrocarbon group is optionally replaced with -0-, -CO-, or -NR¹¹-;
R⁵ represents -OH, -SO₃", -SO₃H, -SO₃~Z⁺, -CO₂H, -C0₂" Z⁺, -CO₂R⁸, -SO₃R⁸, or -SO₂NR⁹R¹⁰;
R⁶ and R⁷ each independently represent a hydrogen atom or an alkyl group having 1 to 6 carbon atoms;
m represents an integer of 0 to 5; when m is 2 or more, a plurality of R⁵ may be the same or different;
a represents an integer of 0 or 1;
X represents a halogen atom;
Z⁺ represents ⁺N (Rⁿ )4, Na⁺, or K⁺, and four R¹¹ may be the same or different;
R⁸ represents a monovalent saturated hydrocarbon group having 1 to 20 carbon atoms, and a hydrogen atom contained in the saturated hydrocarbon group is optionally replaced with a halogen atom;
R⁹ and R¹⁰ each independently represent a hydrogen atom or a monovalent saturated hydrocarbon group having 1 to 20 carbon atoms and optionally having a Substituent, and -CH₂- contained in the saturated hydrocarbon group is optionally replaced with -0-, -CO-, -NH-, or -NR⁸-; and R⁹ and R¹⁰ are optionally bonded to each other to form a 3- to 10-membered heterocycle together with the adjacent nitrogen atom; and
R¹¹ represents a hydrogen atom, a monovalent saturated hydrocarbon group having 1 to 20 carbon atoms, or an aralkyl group having 7 to 10 carbon atoms.

The compound (I) may be a tautomer of the compound. When the compound (I) is used, the content of the compound (I) in the xanthene dye (Al) is preferably 50% by mass or more, more preferably 70% by mass or more, still more preferably 90% by mass or more, and most preferably 100% by mass.

Examples of the monovalent saturated hydrocarbon group having 1 to 20 carbon atoms in R¹ to R⁴ include linear alkyl groups such as a methyl group, an ethyl group, a propyl group, a pentyl group, a hexyl group, a heptyl group, an octyl group, a nonyl group, a decyl group, a dodecyl group, a hexadecyl group, and an icosyl group; branched alkyl groups such as an isopropyl group, an isobutyl group, an isopentyl group, a neopentyl group , and a 2-ethylhexyl group; and alicyclic saturated hydrocarbon groups having 2 to 20 carbon atoms such as a cyclopropyl group, a cyclopentyl group, a cyclohexyl group, a cycloheptyl group, a cyclooctyl group, and a tricyclodecyl group. When the saturated hydrocarbon group has a Substituent, the number of carbon atoms of the saturated hydrocarbon group is the number including the carbon atoms of the Substituent. The Substituent that the saturated hydrocarbon group optionally has is, for example, a halogen atom, -OH, -0R⁸, -S0₃~, -SO₃H, -S0 ₃" Z⁺, -CO₂H, -CO₂R⁸, -SR⁸, -SO₂R⁸, -SO₃R⁸, -SO₂NR⁹R¹⁰, or - Si (OR¹²) (OR¹³) (OR¹⁴) . R¹², R¹³, and R¹⁴ each independently represent a monovalent saturated hydrocarbon group having 1 to 4 carbon atoms, and a hydrogen atom contained in the saturated hydrocarbon group is optionally replaced with a halogen atom.

Examples of the monovalent aromatic hydrocarbon group having 6 to 20 carbon atoms in R¹ to R⁴ include a monocyclic aromatic hydrocarbon group such as a phenyl group, and a polycyclic aromatic hydrocarbon group such as a naphthyl group, an anthryl group, a phenanthryl group, a biphenyl group, or a terphenyl group. The polycyclic aromatic hydrocarbon group is preferably a non-condensed polycyclic aromatic hydrocarbon group such as a biphenyl group or a terphenyl group. The polycyclic aromatic hydrocarbon group preferably does not have a substituent. Examples of the monovalent aromatic hydrocarbon group having a substituent include a toluyl group, a xylyl group, a mesityl group, a propylphenyl group, and a butylphenyl group. When the aromatic hydrocarbon group has a substituent, the number of carbon atoms of the aromatic hydrocarbon group is the number including the carbon atoms of the Substituent. The substituent that the aromatic hydrocarbon group optionally has is, for example, a halogen atom, -R⁸, -OH, -OR⁸, -SO₃-, -SO3H, -SO₃~Z⁺, -CO₂H, -CO₂R⁸, -SR⁸, -SO₂R⁸, - SO3R⁸, -SO₂NR⁹R¹⁰, or -Si(OR¹²) (OR¹³) (OR¹⁴) .

Examples of the monovalent saturated hydrocarbon group having 1 to 20 carbon atoms in R⁸ to R¹¹ include linear alkyl groups such as a methyl group, an ethyl group, a propyl group, a butyl group, a pentyl group, a hexyl group, a heptyl group, an octyl group, a nonyl group, a decyl group, a dodecyl group, a hexadecyl group, and an icosyl group; branched alkyl groups such as an isopropyl group, an isobutyl group, an isopentyl group, a neopentyl group, and a 2-ethylhexyl group; and alicyclic saturated hydrocarbon groups having 3 to 20 carbon atoms such as a cyclopropyl group, a cyclopentyl group, a cyclohexyl group, a cycloheptyl group, a cyclooctyl group, and a tricyclodecyl group.

The monovalent saturated hydrocarbon group having 1 to 20 carbon atoms in R⁹ and R¹⁰ may have a substituent. Examples of the substituent include a hydroxy group and a halogen atom.

Examples of the monovalent saturated hydrocarbon group having 1 to 4 carbon atoms in R¹² to R¹⁴ include linear alkyl groups such as a methyl group, an ethyl group, a propyl group, and a butyl group; and alicyclic saturated hydrocarbon groups having 1 to 4 carbon atoms, for example, branched alkyl groups such as an isopropyl group and an isobutyl group.

Z⁺ is ⁺N(R¹¹)₄, Na⁺, or K⁺, preferably ⁺N(Rⁿ)₄. At least two of four R¹¹ in ⁺N(R¹¹)₄ are preferably monovalent saturated hydrocarbon groups having 5 to 20 carbon atoms. The total number of carbon atoms of four R¹¹ is preferably 20 to 80, and more preferably 20 to 60. When ⁺N(R¹¹)₄ is present in the compound (I), and R¹¹ are these groups, a color filter having less foreign matters can be formed from the negative resist composition of the present invention containing the compound (I).

Examples of -OR⁸ include a methoxy group, an ethoxy group, a propoxy group, a butoxy group, a pentyloxy group, a hexyloxy group, a heptyloxy group, an octyloxy group, a 2-ethylhexyloxy group, and an icosyloxy group.

Examples of -CO2R⁸ include a methoxycarbonyl group, an ethoxycarbonyl group, a propoxycarbonyl group, a tert-butoxycarbonyl group, a hexyloxycarbonyl group, and an icosyloxycarbonyl group.

Examples of -SR⁸ include a methylsulfanyl group, an ethylsulf anyl group, a butylsulfanyl group, a hexylsulfanyl group, a decylsulfanyl group, and an icosylsulfanyl group.

Examples of -SO2R⁸ include a methylsulfonyl group, an ethylsulf onyl group, a butylsulfonyl group, a hexylsulf onyl group, a decylsulf onyl group, and an icosylsulfonyl group.

Examples of -SO3R⁸ include a methoxysulf onyl group, an ethoxysulfonyl group, a propoxysulfonyl group, a tert-butoxysulfonyl group, a hexyloxysulfonyl group, and an icosyloxysulfonyl group.

Examples of -SO₂NR⁹R¹⁰ include a sulfamoyl group;
N-l-substituted sulfamoyl groups such as an N-methylsulfamoyl group, an N-ethylsulf amoyl group, an N-propylsulf amoyl group, an N-isopropylsulf amoyl group, an N-butylsulfamoyl group, an N-isobutylsulf amoyl group, an N-sec-butylsulfamoyl group, an N-tert-butylsulfamoyl group, an N-pentylsulf amoyl group, an N-(l-ethylpropyl) sulfamoyl group, an N-(l,l-dimethylpropyl) sulfamoyl group, an N-(l,2-dimethylpropyl ) sulf amoyl group, an N-(2,2-dimethylpropyl)sulfamoyl group, an N-(l-methylbutyl)sulfamoyl group, an N-(2-methylbutyl) sulfamoyl group, an N-{3-methylbutyl)sulfamoyl group, an N-cyclopentylsulfamoyl group, an N-hexylsulfamoyl group, an N-(l,3-dimethylbutyl)sulfamoyl group, an N-(3,3-dimethylbutyl)sulfamoyl group, an N-heptylsulfamoyl group, an N- (1-methylhexyl) sulfamoyl group, an N-(l,4-dimethylpentyl)sulfamoyl group, an N-octylsulfamoyl group, an N- (2-ethylhexyl)sulfamoyl group, an N-(l,5-dimethyl)hexylsulfamoyl group, and an N- (1,1,2,2-tetramethylbutyl)sulfamoyl group; and
N,N-2-substituted sulfamoyl groups such as an N,N-dimethylsulfamoyl group, an N,N-ethylmethylsulfamoyl group, an N,N-dietmylsulfamoyl group, an N,N-propylmethylsulfamoyl group, an N,N-isopropylmethylsulfamoyl group, an N,N-tert-butylmethylsulfamoyl group, an N,N-butylethylsulfamoyl group, an N,N-bis(1-methylpropyl)sulfamoyl group, and an N,N-heptylmethylsulfamoyl group.

Examples of -Si(OR¹²)(OR¹³)(OR¹⁴) include a trimethoxysilyl group and a triethoxysilyl group.

R⁵ is preferably -CO₂H, -CO₂"Z⁺, -CO₂R⁸, -S0₃", -SO₃'Z⁺, -SO₃H, or SO₂NHR⁹, and more preferably SO₃~, -SO₃~Z⁺, -SO₃H, or SO₂NHR⁹.
m represents an integer of 0 to 5, and is preferably 1 to 4, more preferably 1 or 2, and still more preferably 1.

Examples of the alkyl group having 1 to 6 carbon atoms in R⁶ and R⁷ include the alkyl groups having 1 to 6 carbon atoms among the above-mentioned alkyl groups, with an alkyl group having 1 or 2 carbon atoms being preferred. R⁶ and R⁷ are more preferably hydrogen atoms.

Examples of the aralkyl group having 7 to 10 carbon atoms in R¹¹ include a benzyl group, a phenylethyl group, and a phenylbutyl group.

Examples of the halogen atom in X include a chlorine atom, a fluorine atom, and a bromine atom.
a represents an integer of 0 or 1, and is preferably 0 .

The compound (I) is preferably a compound represented by formula (la) (hereinafter "compound (la)", for example. The compound represented by formula (la) may be used without combination with a compound other than the compound (la) of the compounds (I) (hereinafter sometimes referred to as "compound (Ib)"), or the compound (la) may be used in combination with the compound (Ib) . Alternatively, two or more of the compounds (la) may be used in combination. 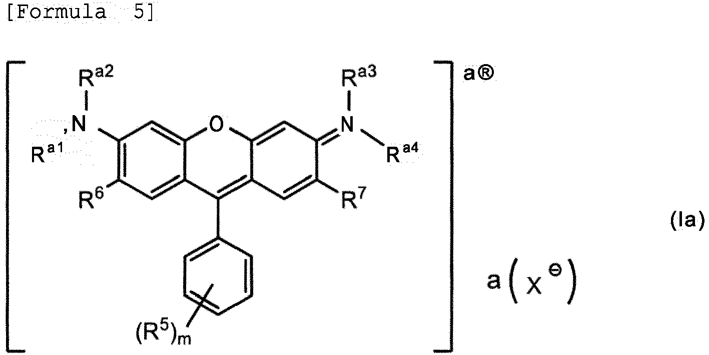 wherein:
R^{al} and R^{a4} are each independently a monovalent aromatic hydrocarbon group optionally having two or less monovalent saturated aliphatic hydrocarbon groups having 1 to 4 carbon atoms;
R^{a2} and R^{a3} are each independently a hydrogen atom, a methyl group, or an ethyl group; and
R⁵ to R⁷, m, a, and X represent the same meanings as the above.

Examples of R^{al} and R^{a4} include, among the same groups as those of R¹ and R⁴ above, a monovalent aromatic hydrocarbon group not having a Substituent or a monovalent aromatic hydrocarbon group having two or less monovalent saturated aliphatic hydrocarbon groups having 1 to 4 carbon atoms. Among these, preferred is a monovalent aromatic hydrocarbon group having two or less monovalent saturated aliphatic hydrocarbon groups having 1 to 4 carbon atoms.

Examples of the monovalent aromatic hydrocarbon group not having a substituent include a monocyclic aromatic hydrocarbon group such as a phenyl group, and a polycyclic aromatic hydrocarbon group such as a naphthyl group, an anthryl group, a phenanthryl group, a biphenyl group, or a terphenyl group. The polycyclic aromatic hydrocarbon group is preferably a non-condensed polycyclic aromatic hydrocarbon group such as a biphenyl group or a terphenyl group. Examples of the monovalent aromatic hydrocarbon group having two or less monovalent saturated aliphatic hydrocarbon groups having 1 to 4 carbon atoms include a toluyl group, a xylyl group, a mesityl group, a monopropylphenyl group, a dipropylphenyl group, a monobutylphenyl group, and a dibutylphenyl group. The number of carbon atoms of the aromatic hydrocarbon group is preferably 7 to 20, more preferably 7 to 16, still more preferably 7 to 10, and most preferably 8. The number of carbon atoms of the aromatic hydrocarbon group is the number including the carbon atoms of the substituents . The aromatic hydrocarbon group preferably does not have a substituent other than the saturated aliphatic hydrocarbon groups.

The number of the saturated aliphatic hydrocarbon groups bonded to the aromatic hydrocarbon group is preferably 1 or 2, and more preferably 2. The saturated aliphatic hydrocarbon groups are preferably bonded to the ortho- or meta-position, more preferably the ortho-position, relative to the point of attachment of the aromatic hydrocarbon group. Examples of the saturated aliphatic hydrocarbon groups include saturated aliphatic hydrocarbon groups not having a Substituent. The number of carbon atoms of the saturated aliphatic hydrocarbon groups is preferably 1 to 4, more preferably 1 to 3, still more preferably 1 or 2, and most preferably 1.

Examples of R^{a2} and R^{a3} include a hydrogen atom, a methyl group, and an ethyl group. Among these, a hydrogen atom or a methyl group is preferred, and a hydrogen atom is more preferred.

The compound (Ib) is preferably a compound represented by formula (Ibl) (hereinafter sometimes referred to as "compound (Ibl)") . While the compound formula (Ibl) is preferably used in combination with the compound (la), it may be used without combination with the compound (la). That is, the xanthene dye (Al) may be the compound (la) and/or the compound (Ibl), is preferably the compound (la), or the compound (la) and the compound (Ibl), and more preferably the compound (la) . 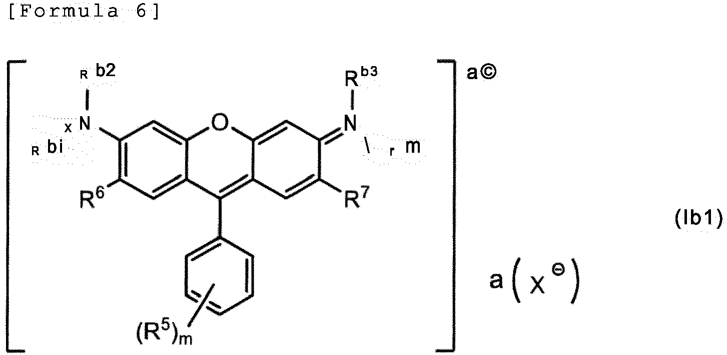
wherein R^{bl} to R^{b4} each independently represent a hydrogen atom, a monovalent saturated hydrocarbon group having 1 to 20 carbon atoms and optionally having a Substituent, or a monovalent aromatic hydrocarbon group having 6 to 20 carbon atoms and optionally having a Substituent;
at least one saturated hydrocarbon group or aromatic hydrocarbon group contained in R^{bl} to R^{b4} has a halogen atom, -OH, -OR⁸, -CO₂H, -CO₂R⁸, -SO₃", -SO₃H, -SO₃~Z⁺, -SR⁸, -SO₂R⁸, -SO₃R⁸, -SO₂NR⁹R¹⁰, or -Si(OR¹²)(OR¹³)(OR¹⁴) as a Substituent, or at least one aromatic hydrocarbon group contained in R^{bl} to R^{b4} has three or more monovalent saturated aliphatic hydrocarbon groups having 1 to 4 carbon atoms as substituents;
R¹², R¹³, and R¹⁴ each independently represent a monovalent saturated hydrocarbon group having 1 to 4 carbon atoms, and a hydrogen atom contained in the saturated hydrocarbon group is optionally replaced with a halogen atom; and
R⁵ to R¹⁰, m, a, and X represent the same meanings as the above .

Examples of R^{bl} and R^{b4} include, among the same groups as those of R¹ and R⁴ above, a hydrogen atom, a monovalent saturated hydrocarbon group having 1 to 20 carbon atoms and optionally having a Substituent, a monovalent aromatic hydrocarbon group having 6 to 20 carbon atoms and optionally having a Substituent, or an aromatic hydrocarbon group having three or more monovalent saturated aliphatic hydrocarbon groups having 1 to 4 carbon atoms as substituents. Among these, a monovalent saturated hydrocarbon group having 1 to 20 carbon atoms and optionally having a substituent, a monovalent aromatic hydrocarbon group having 6 to 20 carbon atoms and having a substituent, or an aromatic hydrocarbon group having three or more monovalent saturated aliphatic hydrocarbon groups having 1 to 4 carbon atoms as substituents is preferred.

The number of carbon atoms of the monovalent saturated hydrocarbon group having 1 to 20 carbon atoms and optionally having a substituent is preferably 1 to 10, more preferably 2 to 8, and still more preferably 2 to 7. The number of carbon atoms of the saturated aliphatic hydrocarbon group is the number including the carbon atoms of the Substituent. The Substituent is preferably a halogen atom, -OH, -0R⁸, -CO₂H, -CO₂R⁸, -S0₃-, -SO₃H, -SO₃"Z⁺, -SR⁸, -SO₂R⁸, -SO₃R⁸, -SO₂NR⁹R¹⁰, or - Si(OR¹²)(OR¹³)(OR¹⁴), more preferably -OH, -OR⁸, -CO₂H, - CO₂R⁸, -S0 ₃-, -SO₃H, -SO₃~Z⁺, -SR⁸, -SO₂R⁸, -SO₃R⁸, or - Si (OR¹²) (OR¹³) (OR¹⁴), still more preferably -OH, -OR⁸, - CO₂H, -CO₂R⁸, or -Si(OR¹²) (OR¹³) (OR¹⁴), and most preferably -Si(OR¹²) (OR¹³) (OR¹⁴). The number of substituents is preferably 1 to 5, more preferably 1 to 3, still more preferably 1 or 2, and most preferably 1, in one saturated aliphatic hydrocarbon group.

The number of carbon atoms of the monovalent aromatic hydrocarbon group having 6 to 20 carbon atoms and optionally having a Substituent is preferably 7 to 20, more preferably 7 to 16, still more preferably 7 to 12, even more preferably 7 to 10, particularly preferably 7 or 8, and most preferably 8. The number of carbon atoms of the aromatic hydrocarbon group is the number including the carbon atoms of the Substituent. The substituent is preferably a monovalent saturated aliphatic hydrocarbon group having 1 to 4 carbon atoms, a halogen atom, -OH, -OR⁸, -CO₂H, -CO₂R⁸, -SO₃", -SO₃H, -SO₃~ Z⁺, -SR⁸, -SO₂R⁸, -SO₃R⁸, -SO₂NR⁹R¹⁰, or - Si(OR¹²)(OR¹³)(OR¹⁴), and more preferably a monovalent saturated aliphatic hydrocarbon group having 1 to 4 carbon atoms, -OH, -OR⁸, -CO₂H, -CO₂R⁸, -S0₃~, -SO₃H, -S0₃" Z⁺, -SR⁸, -SO₂R⁸, -SO₃R⁸, or -SO₂NR⁹R¹⁰. The Substituent is still more preferably a monovalent saturated aliphatic hydrocarbon group having 1 to 4 carbon atoms, -OR⁸, -S0 ₃-, -SO₃H, -SO₃-Z⁺, -SR⁸, -SO₂R⁸, -SO₃R⁸, or -SO₂NR⁹R¹⁰. The number of substituents is preferably 1 to 5, more preferably 1 to 3, still more preferably 1 or 2, and most preferably 2, in one aromatic hydrocarbon group. The Substituent is preferably bonded to the ortho- and/or meta-position, more preferably the ortho-position, relative to the point of attachment of the aromatic hydrocarbon group.

The number of carbon atoms of the aromatic hydrocarbon group having three or more monovalent saturated aliphatic hydrocarbon groups having 1 to 4 carbon atoms as substituents is preferably 9 to 20, more preferably 9 to 13, still more preferably 9 to 12, and most preferably 9. The number of carbon atoms of the aromatic hydrocarbon group is the number including the carbon atoms of the substituents. The aromatic hydrocarbon group preferably does not have a Substituent other than the saturated aliphatic hydrocarbon groups. The number of the saturated aliphatic hydrocarbon groups is preferably 3 to 5, more preferably 3 or 4, and most preferably 3, in one aromatic hydrocarbon group. The saturated aliphatic hydrocarbon groups are preferably bonded to the ortho- and/or para-position, more preferably the ortho- and para-positions, relative to the point of attachment of the aromatic hydrocarbon group . The number of carbon atoms of the monovalent saturated aliphatic hydrocarbon groups is preferably 1 to 5, more preferably 1 to 3, still more preferably 1 or 2, and most preferably 1.

Preferred as R^{b2} and R^{b3} is a hydrogen atom or a monovalent saturated hydrocarbon group having 1 to 20 carbon atoms and optionally having a Substituent, for example, among the same groups as those of R² and R³ above. Among these, a monovalent saturated hydrocarbon group having 1 to 20 carbon atoms and optionally having a Substituent is more preferred, and a monovalent saturated hydrocarbon group having 1 to 20 carbon atoms and having a Substituent is still more preferred.

The number of carbon atoms of the monovalent saturated hydrocarbon group having 1 to 20 carbon atoms and optionally having a Substituent is preferably 1 to 10, more preferably 1 to 6, and still more preferably 1 to 4. The number of carbon atoms of the saturated aliphatic hydrocarbon group is the number including the carbon atoms of the Substituent. The Substituent is preferably a halogen atom, -OH, -OR⁸' -CO2H, -CO2R⁸, -SO3", -SO₃H, -SO₃~Z⁺, -SR⁸, -SO₂R⁸, -SO₃R⁸, -SO₂NR⁹R¹⁰, or - Si (OR¹²) (OR¹³) (OR¹⁴), for example, more preferably -OH, - OR⁸, -CO₂H, -CO2R⁸, -SO₃", -SO₃H, -SO₃~Z⁺, -SR⁸, -SO₂R⁸, - SO₃R⁸, or -Si(OR¹²) (OR¹³) (OR¹⁴), still more preferably -OH, -OR⁸, -CO₂H, -CO2R⁸, or -Si (OR¹²) (OR¹³) (OR¹⁴), and most preferably -CO₂H or -CO₂R⁸. The number of substituents is preferably 1 to 5, more preferably 1 to 3, still more preferably 1 or 2, and most preferably 1, in one saturated aliphatic hydrocarbon group.

Preferred as the compound (la) are compounds No. 1 to 48 specified by formula (laX) and in Table 1.

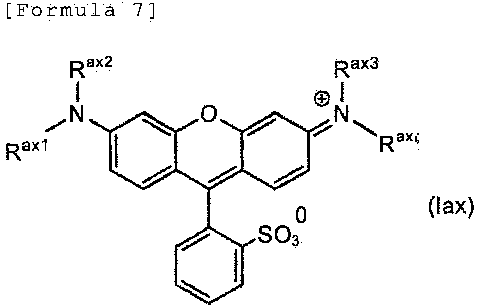

**[Table 1]**

| **No.** | Rax1 | Rax2 | Rax3 | Rax4 | **No.** | Rax1 | Rax2 | Rax3 | Rax4 |
|---|---|---|---|---|---|---|---|---|---|
| **1** | **Ph** | H | H | **Ph** | **25** | **BPh2** | H | H | **BPh2** |
| **2** | **Ph** | CH₃ | **ch₃** | **Ph** | **26** | **BPh2** | **ch₃** | CH3 | **BPh2** |
| **3** | **Ph** | CH3CH2 | CH3CH2 | **Ph** | **27** | **BPh2** | CH3CH2 | CH3CH2 | **BPh2** |
| **4** | **o-Tolyl** | H | H | **o-Tolyl** | **28** | **BPh3** | H | H | **BPh3** |
| **5** | **o-Tolyl** | CHs | **ch₃** | **o-Tolyl** | **29** | **BPh3** | **ch₃** | **ch₃** | **BPh3** |
| **6** | **o-Tolyl** | CH3CH2 | CH3CH2 | o-Tolyl | 30 | BPh3 | CH3CH2 | CH3CH2 | BPh3 |
| **7** | m-Tolyl | H | H | m-Tolyl | 31 | TPh2' | H | H | 2,6-Xylyl |
| **8** | m-Tolyl | CH3 | **ch₃** | m-Tolyl | 32 | TPh2' | **ch₃** | **ch₃** | 2,6-Xylyl |
| **9** | m-Tolyl | CH3CH2 | CH3CH2 | m-Tolyl | 33 | TPh2' | CH3CH2 | CH3CH2 | 2,6-Xylyl |
| **10** | p-Tolyl | H | H | p-Tolyl | 34 | TPh2' | H | H | BPh2 |
| **11** | p-Tolyl | **ch₃** | CH ₃ | p-Tolyl | 35 | TPh2' | **ch₃** | CH₃ | BPh2 |
| **12** | p-Tolyl | CH3CH2 | CH3CH2 | p-Tolyl | 36 | TPh2' | **ch₃ch₂** | **ch₃ch₂** | BPh2 |
| **13** | 2,6-Xylyl | H | H | 2,6-Xylyl | 37 | TPh2' | H | H | BPh3 |
| **14** | 2,6-Xylyl | **ch₃** | CH₃ | 2,6-Xylyl | 38 | TPh2' | CH₃ | **ch₃** | BPh3 |
| **15** | 2,6-Xylyl | CH3CH2 | CH3CH2 | 2,6-Xylyl | 39 | TPh2' | **ch₃ch₂** | CH₃CH2 | BPh3 |
| **16** | 2,6-Xylyl | H | H | Ph | 40 | TPh2' | H | H | TPh2' |
| **17** | 2,6-Xylyl | CH ₃ | CH₃ | Ph | 41 | TPh2' | CH ₃ | **ch₃** | TPh2' |
| **18** | 2,6-Xylyl | CH3CH2 | CH3CH2 | Ph | 42 | TPh2' | CH3CH2 | CH₃CH2 | TPh2' |
| **19** | 2,6-Xylyl | H | H | BPh2 | 43 | p-ButylPh | H | H | p-ButylPh |
| **20** | 2,6-Xylyl | CH₃ | **ch₃** | BPh2 | 44 | p-ButylPh | CH₃ | **ch₃** | p-ButylPh |
| **21** | 2,6-Xylyl | CH3CH2 | CH3CH2 | BPh2 | 45 | p-ButylPh | **ch₃ch₂** | CH3CH2 | p-ButylPh |
| **22** | 2,6-Xylyl | H | H | BPh3 | 48 | m-ButylPh | H | H | m-ButylPh |
| **23** | 2,6-Xylyl | CH₃ | CH₃ | BPh3 | 47 | m-ButylPh | CH₃ | **ch₃** | m-ButylPh \| |
| **24** | 2,6-Xylyl | CH3CH2 | CH3CH2 | BPh3 | 48 | m-ButylPh | CH3CH2 | **ch₃ch₂** | m-ButylPh |

The Symbols in the formula mean the following groups (hereinafter, * represents a point of attachment) :

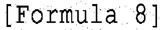

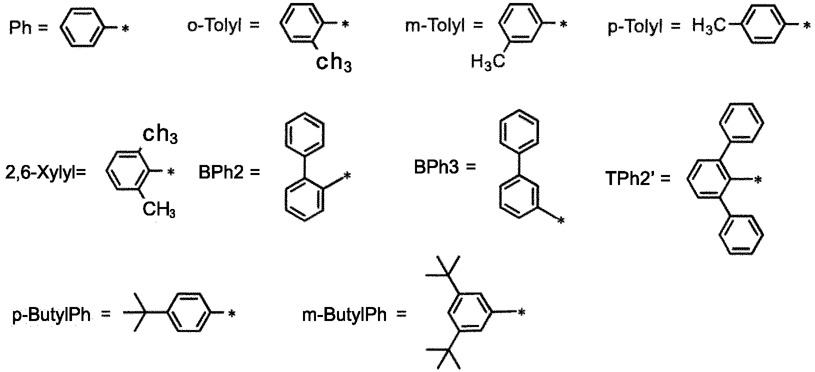

Preferred as the compound (Ibl) are compounds No. 49 to 74 specified by formula (Ibx) and in Table 2, and compounds represented by formulae A3-1 to A3-8.

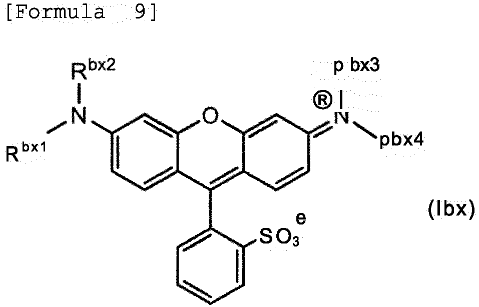

**[Table 2]**

| No. | Rbx1 | Rbx2 | Rbx3 | Rbx4 |
|---|---|---|---|---|
| 49 | ch₃ | ch₃ | ch₃ | PrTMS |
| 50 | CH3CH2 | ch₃ch₂ | ch₃ | PrTMS |
| 51 | CH3CH2 | CH3CH2 | CH3CH2 | PrTMS |
| 52 | Ph | PrCOOH | PrCOOH | Ph |
| 53 | o-Tolyl | PrCOOH | PrCOOH | o-Tolyl |
| 54 | m-Tolyl | PrCOOH | PrCOOH | m-Tolyl |
| 55 | p-Tolyl | PrCOOH | PrCOOH | p-Tolyl |
| 56 | 2,6-Xylyl | PrCOOH | PrCOOH | 2,6-Xylyl |
| 57 | MT1 | H | H | MT1 |
| 58 | MT1 | CH₃ | CH3 | MT1 |
| 59 | MT1 | CH3CH2 | CH3CH2 | MT1 |
| 60 | MT2 | H | H | MT2 |
| 61 | MT2 | CH3 | ch₃ | MT2 |
| 62 | MT2 | CH3CH2 | CH3CH2 | MT2 |
| 63 | MT3 | H | H | MT3 |
| 64 | MT3 | CH3 | ch₃ | MT3 |
| 65 | MT3 | CH3CH2 | CH3CH2 | MT3 |
| 66 | 2,4,6-Mesityl | H | H | 2,4,6-Mesityl |
| 67 | 2,4,6-Mesityl | CH₃ | CH3 | 2,4,6-Mesityl |
| 68 | 2,4,6-Mesityl | CH3CH2 | CH3CH2 | 2,4,6-Mesityl |
| 69 | 2,6-Xylyl | H | H | MPh1 |
| 70 | 2,6-Xylyl | CH3 | CH3 | MPh1 |
| 71 | 2,6-Xylyl | CH3CH2 | CH3CH2 | MPh1 |
| 72 | 2,6-Xylyl | H | H | MPh2 |
| 73 | 2,6-Xylyl | ch₃ | CH ₃ | MPh2 |
| 74 | 2,6-Xylyl | CH3CH2 | CH3CH2 | MPh2 |

The Symbols in the formula mean the following groups (hereinafter, * represents a point of attachment) :

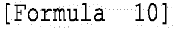

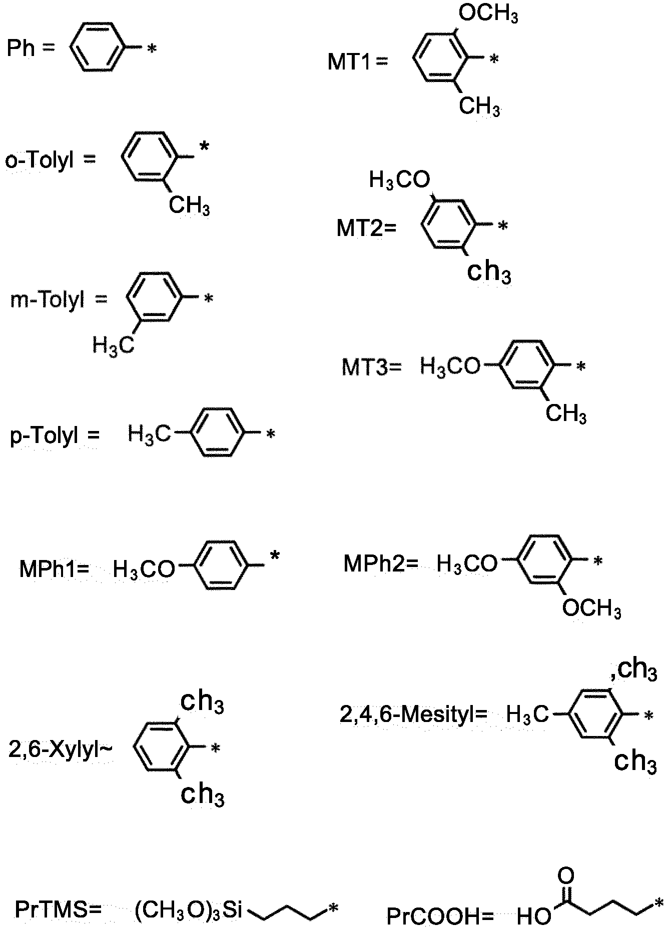

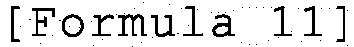

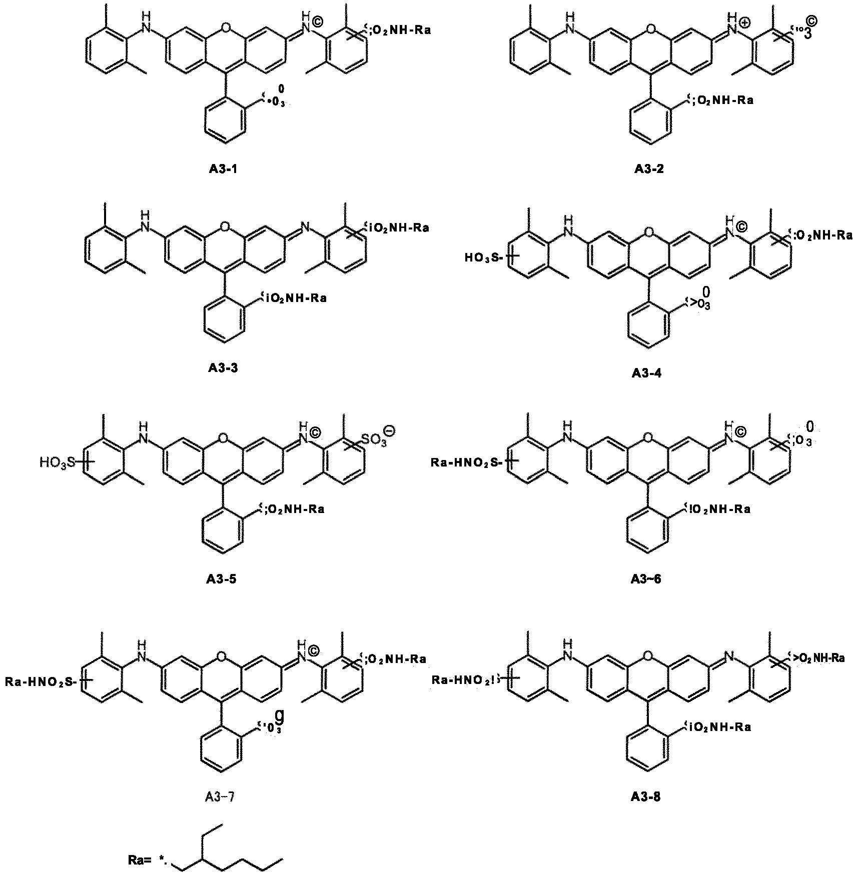

The colorant (A) may be a dye alone or a combination of a dye and a pigment. Among these, the colorant (A) is preferably a dye alone or a combination of a dye and a red pigment or a purple pigment, more preferably the xanthene dye (Al) alone or a combination of the xanthene dye (Al) and a red pigment or a purple pigment, still more preferably the xanthene dye (Al) alone or a combination of the xanthene dye (Al) and a purple pigment, and even more preferably the xanthene dye (Al) alone .

The xanthene dye (Al) is preferably the compound (la) and/or the compound (Ibl). That is, the xanthene dye (Al) is preferably either the compound (la) or compound (Ibl), or a combination of the compound (la) and the compound (Ibl).

The xanthene dye (Al) is also preferably a combination of two or more selected from the compounds (lb), or a combination of the compound (Ib) and the compound (la). More preferably, the xanthene dye (Al) is a combination of two or more selected from the compounds (Ibl), or a combination of the compound (Ibl) and the compound (la).

The content of the xanthene dye (Al) is preferably 30 to 100% by mass, more preferably 60 to 100% by mass, still more preferably 80 to 100% by mass, and most preferably 100% by mass, relative to the whole amount of the colorant (A). When the colorant (A) contains a pigment and the like, the content of the xanthene dye (Al) may be 90% by mass or less, 80% by mass or less, or 70% by mass or less.

The content of the compound (la) in the xanthene dye (Al) is preferably 30 to 100% by mass, more preferably 60 to 100% by mass, still more preferably 80 to 100% by mass, and most preferably 100% by mass. When the colorant (A) contains the compound (Ib) and the like, the content of the compound (la) may be 80% by mass or less, 70% by mass or less, or 60% by mass or less.

The content of the compound (Ib) in the xanthene dye (Al) is preferably 20 to 80% by mass, more preferably 30 to 70% by mass, and still more preferably 40 to 60% by mass.

The content of the compound (Ib) relative to 100 parts by mass of the compound (la) is preferably 20 to 150 parts by mass, more preferably 50 to 130 parts by mass, and still more preferably 80 to 110 parts by mass.

### COther Dye (A2)>

The colorant (A) may further contain a dye other than the xanthene dye (Al) (sometimes referred to as "other dye (A2) ") . The other dye (A2) is not particularly limited as long as it is a dye other than the xanthene dye (Al), and examples thereof include dyes such as an oil-soluble dye, an acid dye, a basic dye, a direct dye, a mordant dye, and an amine salt of the acid dye or a Sulfonamide derivative of the acid dye, for example, a compound classified into a dye in the Color Index (published by The Society of Dyers and Colourists), i.e., a compound with a hue other than C.I. Pigment, and a known dye as described in Dying note (Shikisensha Co., Ltd.). According to Chemical structures, examples thereof include an azo dye, an anthraquinone dye, a cyanine dye, a phthalocyanine dye, a naphthoquinone dye, a quinonimine dye, a methine dye, an azomethine dye, a squarylium dye, an acridine dye, a styryl dye, a coumarin dye, a quinoline dye, and a nitro dye.

### <Pigment (A3)>

The colorant (A) may contain a pigment (A3). Examples of the pigment (A3) include an organic pigment and an inorganic pigment, and examples thereof include a compound classified into a pigment in the Color Index (published by The Society of Dyers and Colourists).

Examples of the organic pigment include red pigments such as C.I. Pigment Reds 9, 97, 105, 122, 123, 144, 149, 166, 168, 176, 177, 180, 192, 209, 215, 216, 224, 242, 254, 255, 264, and 265;
blue pigments such as C.I. Pigment Blues 15, 15:3, 15:4, 15:6, and 60; violet pigments such as C.I. Pigment Violets 1, 19, 23, 29, 32, 36, and 38; and
green pigments such as C.I. Pigment Greens 7, 36, and 58.

Among these, a red pigment and a purple pigment are preferred, a purple pigment is more preferred, and C.I. Pigment Violet 19 is still more preferred.

Examples of the inorganic pigment include metal compounds such as metal oxides or metal complex salts, and specific examples include oxides or composite metal oxides of metals such as iron, cobalt, aluminum, cadmium, lead, copper, titanium, magnesium, chromium, zinc, and antimony .

The pigment (A3) may he subjected to a rosin treatment, a surface treatment using a pigment derivative, a dispersant or the like having an introduced acidic group or basic group, a pigment surface graft treatment with a polymeric compound or the like, a particle micronization treatment with a sulfuric acid micronization method or the like, a washing treatment with an organic solvent, water or the like for removing impurities, a removing treatment with an ionic exchange method or the like of ionic impurities or the like, if required. The pigment (A3) preferably has a uniform particle diameter.

When the pigment (A3) is used, the content of the pigment (A3) is preferably 10 to 90% by mass, more preferably 20 to 80% by mass, and still more preferably 30 to 70% by mass, relative to the whole amount of the colorant (A).

### <Solvent (B)>

The negative resist composition may contain a solvent (B). The solvent (B) is not particularly limited, and a solvent commonly used in the art can be used. For example, a solvent selected from an ester solvent (a solvent containing -COO- and not containing - 0- in its molecule), an ether solvent (a solvent containing -0- and not containing -COO- in its molecule), an ether ester solvent (a solvent containing -COO- and - 0- in its molecule), a ketone solvent (a solvent containing -CO- and not containing -COO- in its molecule), an alcohol solvent (a solvent containing OH and not containing -0-, -CO-, and -COO- in its molecule), an aromatic hydrocarbon solvent, an amide solvent, dimethyl sulfoxide and the like can be used.

Examples of the ester solvent include methyl lactate, ethyl lactate, butyl lactate, methyl 2-hydroxyisobutanoate, ethyl acetate, n-butyl acetate, isobutyl acetate, pentyl formate, isopentyl acetate, butyl propionate, isopropyl butyrate, ethyl butyrate, butyl butyrate, methyl pyruvate, ethyl pyruvate, propyl pyruvate, methyl acetoacetate, ethyl acetoacetate, cyclohexanol acetate, y-butyrolactone, propylene glycol diacetate, and 1,3-butylene glycol diacetate.

Examples of the ether solvent include ethylene glycol monoalkyl ethers such as ethylene glycol monomethyl ether, ethylene glycol monoethyl ether, ethylene glycol monopropyl ether, and ethylene glycol monobutyl ether; diethylene glycol monoalkyl ethers such as diethylene glycol monomethyl ether, diethylene glycol monoethyl ether, and diethylene glycol monobutyl ether; propylene glycol monoalkyl ethers such as propylene glycol monomethyl ether, propylene glycol monoethyl ether, propylene glycol monopropyl ether, and propylene glycol monobutyl ether; cyclic ethers such as tetrahydrofuran, tetrahydropyran, and 1,4-dioxane; diethylene glycol dialkyl ethers such as diethylene glycol dimethyl ether, diethylene glycol diethyl ether, diethylene glycol ethyl methyl ether, diethylene glycol dipropyl ether, and diethylene glycol dibutyl ether; dipropylene glycol dialkyl ethers such as dipropylene glycol dimethyl ether; phenol ethers such as anisole, phenetol, and methyl anisole; 3-methoxy-l-butanol, and 3-methoxy-3-methylbutanol .

Examples of the ether ester solvent include ethylene glycol monoalkyl ether acetates such as ethylene glycol monomethyl ether acetate and ethylene glycol monoethyl ether acetate; propylene glycol monoalkyl ether acetates such as propylene glycol monomethyl ether acetate, propylene glycol monoethyl ether acetate, and propylene glycol monopropyl ether acetate; diethylene glycol monoalkyl ether acetates such as diethylene glycol monoethyl ether acetate and diethylene glycol monobutyl ether acetate; dipropylene glycol monoalkyl ether acetates such as dipropylene glycol monomethyl ether acetate; methyl methoxyacetate, ethyl methoxyacetate, butyl methoxyacetate, methyl ethoxyacetate, ethyl ethoxyacetate, methyl 3-methoxypropionate, ethyl 3-methoxypropionate, methyl 3-ethoxypropionate , ethyl 3-ethoxypropionate , methyl 2-methoxypropionate, ethyl 2-methoxypropionate, propyl 2-methoxypropionate, methyl 2-ethoxypropionate, ethyl 2-ethoxypropionate, methyl 2-methoxy-2-methylpropionate, ethyl 2-ethoxy-2-methylpropionate, 3-butyl methoxyacetate, and 3-methyl-3-butyl methoxyacetate.

Examples of the ketone solvent include 4-hydroxy-4-methyl-2-pentanone, acetone, 2-butanone, 2-heptanone, 3-heptanone, 4-heptanone, 4-methyl-2-pentanone, cyclopentanone, cyclohexanone, and isophorone.

Examples of the alcohol solvent include methanol, ethanol, propanol, butanol, hexanol, cyclohexanol, ethylene glycol, propylene glycol, and glycerin.

Examples of the aromatic hydrocarbon solvent include benzene, toluene, xylene, and mesitylene.

Examples of the amide solvent include N,N-dimethylformamide, N,N-dimethylacetamide, and N-methylpyrrolidone.

Among the above-mentioned solvents, propylene glycol monomethyl ether acetate, propylene glycol monomethyl ether, and/or 4-hydroxy-4-methyl-2-pentanone are/is pref erred.

A solvent containing an ether ester solvent is particularly preferred. In this case, the content of the ether ester solvent is preferably 40% by mass or more and 100% by mass or less, relative to the whole amount of the solvent (B), and is more preferably 40% by mass or more and 99% by mass or less, from the viewpoint of dispersion stability. The lower limit of the content of the ether ester solvent is preferably 60% by mass, and more preferably 70% by mass.

The ether ester solvent is preferably at least one selected from the group consisting of ethylene glycol monoalkyl ether acetates, propylene glycol monoalkyl ether acetates, and diethylene glycol monoalkyl ether acetates, and more preferably a propylene glycol monomethyl ether acetate.

When the solvent (B) is a mixed solvent, the solvent to be combined with the ether ester solvent is preferably at least one selected from the group consisting of an ether solvent and a ketone solvent, and more preferably at least one selected from the group consisting of propylene glycol monomethyl ether and 4-hydroxy-4-methyl-2-pentanone.

The solvent (B) is preferably such a solvent because a color filter having a high brightness can be produced from a negative resist composition prepared from the negative resist composition of the present invention.

The solvent (B) is preferably a solvent having a solubility (23°C) of 5% by mass or less for the colorant (A), and more preferably a solvent having a solubility (23°C) of 0.3 to 3% by mass for the colorant (A) . The solvent (B) is particularly preferably a solvent having a solubility that falls within the above-mentioned ränge for the xanthene dye (Al).

### <Resin (C) >

The negative resist composition of the present invention contains a resin (C) . The resin (C) is preferably an alkali soluble resin. When the negative resist composition contains an alkali soluble resin, the solubility of a non-exposed area in a developing solution is improved. An alkali soluble resin also has excellent compatibility with a dispersant having an amine group to be described later. Examples of the resin (C) include the following resins [Kl] to [K6].
Resin [Kl]: a copolymer of least one (a) selected from the group consisting of an unsaturated carboxylic acid and an unsaturated carboxylic anhydride (hereinafter, sometimes referred to as "(a)") and a monomer (b) having a cyclic ether structure having 2 to 4 carbon atoms and an ethylenically unsaturated bond (hereinafter, sometimes referred to as "(b)");
Resin [K2]: a copolymer of (a), (b), and a monomer (c) copolymerizable with (a) (provided that (c) is different from (a) and (b)) (hereinaf ter, sometimes referred to as "(c)");
Resin [K3]: a copolymer of (a) and (c);
Resin [K4] : a resin obtained by reacting the copolymer of (a) and (c) with (b);
Resin [K5]: a resin obtained by reacting a copolymer of (b) and (c) with (a); and
Resin [K6]: a resin obtained by reacting the copolymer of (b) and (c) with (a), and further reacting the resultant product with a carboxylic anhydride.

Specific examples of (a) include unsaturated monocarboxylic acids such as acrylic acid, methacrylic acid, crotonic acid, and o-, m- or p-vinylbenzoic acid;
unsaturated dicarboxylic acids such as maleic acid, fumaric acid, citraconic acid, mesaconic acid, itaconic acid, 3-vinylphthalic acid, 4-vinylphthalic acid, 3, 4, 5, 6-tetrahydrophthalic acid, 1,2,3, 6-tetrahydrophthalic acid, dimethyltetrahydrophthalic acid, and 1,4-cyclohexenedicarboxylic acid;
carboxy group-containing bicyclo unsaturated Compounds such as methyl-5-norbornene-2 , 3-dicarboxylic acid, 5-carboxybicyclo[2.2.1]hept-2-ene, 5,6-dicarboxybicyclo[2.2.1]hept-2-ene, 5-carboxy-5-methylbicyclo[2.2.1] hept-2-ene, 5-carboxy-5-ethylbicyclo[2.2.1] hept-2-ene, 5-carboxy- 6-methylbicyclo[2.2.1]hept-2-ene, and 5-carboxy-6-ethylbicyclo[2.2.1]hept-2-ene;
unsaturated dicarboxylic anhydrides such as maleic anhydride, citraconic anhydride, itaconic anhydride, 3-vinylphthalic anhydride, 4-vinylphthalic anhydride, 3,4,5,6-tetrahydrophthalic anhydride, 1,2,3,6-tetrahydrophthalic anhydride, dimethyltetrahydrophthalic anhydride, and 5,6-dicarboxybicyclo [2.2.1]hept-2-ene anhydride;
unsaturated mono [ (meth) acryloyloxyalkyl] esters of a polyvalent carboxylic acid having a valence of 2 or more such as succinic acid mono[2- (meth)acryloyloxyethyl], and phthalic acid mono[2- (meth)acryloyloxyethyl]; and
unsaturated acrylates containing a hydroxy group and a carboxy group in the same molecule such as a-(hydroxymethyl) acrylic acid.

Among these, from the viewpoint of copolymerization reactivity and solubility of a resin to be produced to an alkaline aqueous solution, acrylic acid, methacrylic acid, maleic anhydride and the like are preferable.
(b) means a polymerizable compound having, for example, a cyclic ether structure having 2 to 4 carbon atoms (for example, at least one selected from the group consisting of an oxirane ring, an oxetane ring, and a tetrahydrofuran ring) and an ethylenically unsaturated bond. (b) is preferably a monomer having a cyclic ether structure having 2 to 4 carbon atoms and a (meth)acryloyloxy group.

As used herein, " (meth) acrylic acid" represents at least one selected from the group consisting of acrylic acid and methacrylic acid. The terms " (meth) acryloyl", "(meth) acrylate" and the like also have similar meanings.

Examples of (b) include a monomer having an oxiranyl group and an ethylenically unsaturated bond (bl) (hereinafter, sometimes referred to as "(bl)"), a monomer having an oxetanyl group and an ethylenically unsaturated bond (b2) (hereinafter, sometimes referred to as "(b2)"), and a monomer having a tetrahydrofuryl group and an ethylenically unsaturated bond (b3) (hereinafter, sometimes referred to as " (b3) ") .

Examples of (bl) include a monomer having a structure where a linear or branched aliphatic unsaturated hydrocarbon is epoxidized (bl-1) (hereinafter, sometimes referred to as "(bl-1)") and a monomer having a structure where an alicyclic unsaturated hydrocarbon is epoxidized (bl-2) (hereinafter, sometimes referred to as "bl-2") .

Examples of (bl-1) include glycidyl(meth)acrylate, β-methylglycidyl (meth) acrylate, β-ethylglycidyl(meth) acrylate, glycidyl vinyl ether, o-vinylbenzyl glycidyl ether, m-vinylbenzyl glycidyl ether, p-vinylbenzyl glycidyl ether, a-methyl-o-vinylbenzyl glycidyl ether, a-methyl-m-vinylbenzyl glycidyl ether, a-methyl-p-vinylbenzyl glycidyl ether, 2,3-bis(glycidyloxymethyl)styrene, 2,4-bis (glycidyloxymethyl)styrene, 2,5-bis(glycidyloxymethyl)styrene, 2,6-bis(glycidyloxymethyl)styrene, 2,3,4-tris (glycidyloxymethyl) styrene, 2, 3, 5-tris(glycidyloxymethyl) styrene, 2,3,6-tris(glycidyloxymethyl)styrene, 3, 4, 5-tris(glycidyloxymethyl)styrene, and 2,4,6-tris(glycidyloxymethyl) styrene.

Examples of (bl-2) include vinylcyclohexene monoxide, 1,2-epoxy-4-vinylcyclohexane (for example, CELLOXIDE (registered trademark) 2000; manufactured by Daicel Corporation), 3,4-epoxycyclohexylmethyl (meth)acrylate (for example, Cyclomer (registered trademark) A400; manufactured by Daicel Corporation), 3,4-epoxycyclohexylmethyl (meth) acrylate (for example, Cyclomer M100; manufactured by Daicel Corporation), a compound represented by formula (BI), and a compound represented by formula (BII) : 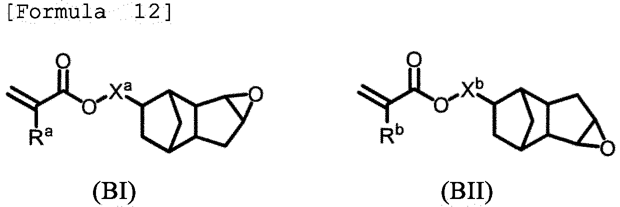 wherein
R^{a} and R^{b} represent a hydrogen atom or an alkyl group having 1 to 4 carbon atoms; the hydrogen atom contained in the alkyl group is optionally replaced with a hydroxy group;
X^{a} and X^{b} represent a single bond, -R^{c}-, *-R^{c}-O-, *-R^{c}-S-, or *-R^{c}-NH-;
R^{c} represents an alkanediyl group having 1 to 6 carbon atoms; and
* represents a point of attachment to 0.

Examples of the alkyl group having 1 to 4 carbon atoms include a methyl group, an ethyl group, a n-propyl group, an isopropyl group, a n-butyl group, a sec-butyl group, and a tert-butyl group.

Examples of the alkyl group in which the hydrogen atom is replaced with the hydroxy group include a hydroxymethyl group, a 1-hydroxyethyl group, a 2-hydroxyethyl group, a 1-hydroxypropyl group, a 2-hydroxypropyl group, a 3-hydroxypropyl group, a 1-hydroxy-l-methylethyl group, a 2-hydroxy-l-methylethyl group, a 1-hydroxybutyl group, a 2-hydroxybutyl group, a 3-hydroxybutyl group, and a 4-hydr oxybutyl group.

Preferably, examples of R^{a} and R^{b} include a hydrogen atom, a methyl group, a hydroxymethyl group, a 1-hydroxyethyl group, and a 2-hydroxyethyl group. More preferably, examples thereof include a hydrogen atom and a methyl group.

Examples of the alkanediyl group include a methylene group, an ethylene group, a propane-1,2-diyl group, a propane-1 ,3-diyl group, a butane-1,4-diyl group, a pentane-1,5-diyl group, and a hexane-1,6-diyl group.

Preferably, examples of X^{a} and X^{b} include a single bond, a methylene group, an ethylene group, *-CH₂-O-, and ★-CH₂CH₂-0-. More preferably, examples thereof include a single bond and *-CH₂CH₂-O- (* represents a point of attachment to 0).

Examples of the compound represented by formula (BI) include a compound represented by formulas (BI-1) to (BI-15). Preferred is the compound represented by formula (BI-1) , formula (BI-3) , formula (BI-5) , formula (BI-7) , formula (BI-9), or formula (BI-11) to formula (BI-15), for example. More preferred is the compound represented by formula (BI-1), formula (BI-7), formula (BI-9), or formula (BI-15), for example.

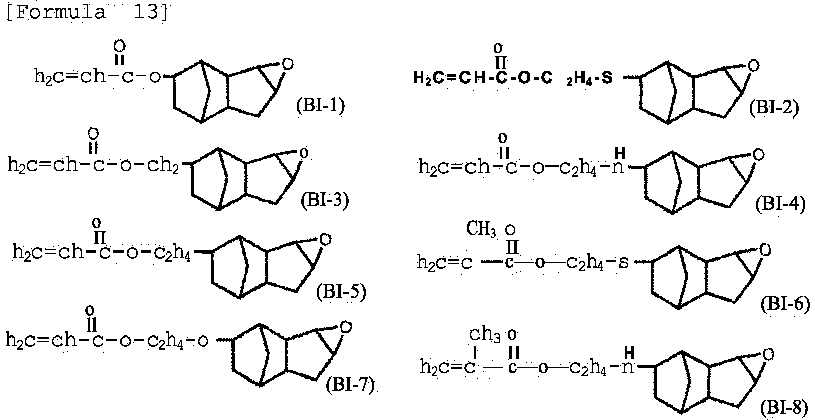

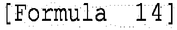

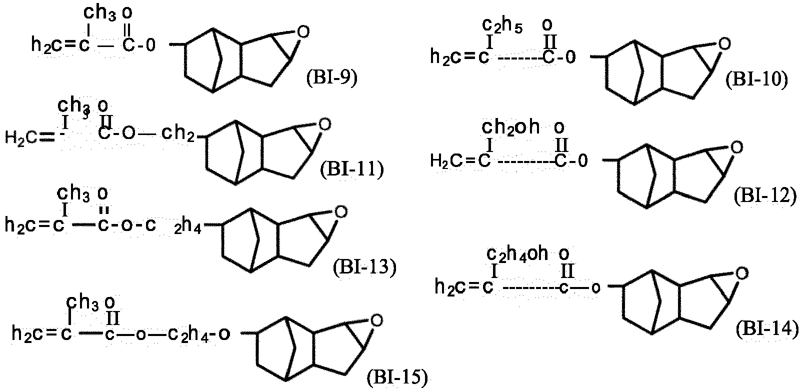

Examples of the compound represented by formula (BIT) include a compound represented by formula (BII-1) to formula (BII-15) . Preferred is the compound represented by formula (BII-1) , formula (BII-3), formula (BII-5), formula (BII-7) , formula (BII-9) , or formula (BII-11) to formula (BII-15), for example. More preferred is the compound represented by formula (BII-1), formula (BII-7), formula (BII-9), or formula (BII-15), for example.

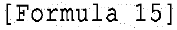

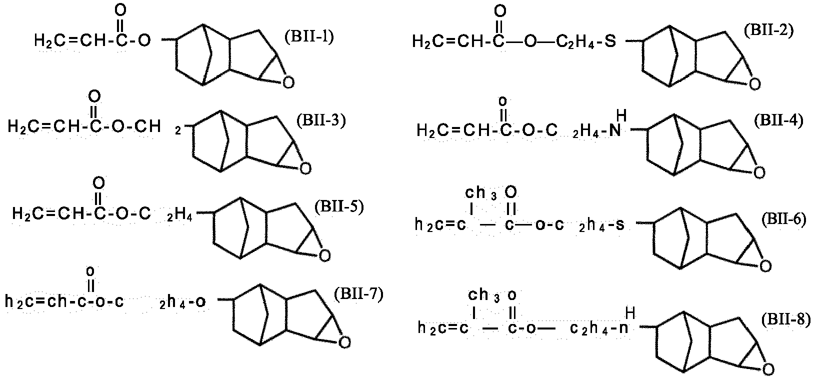

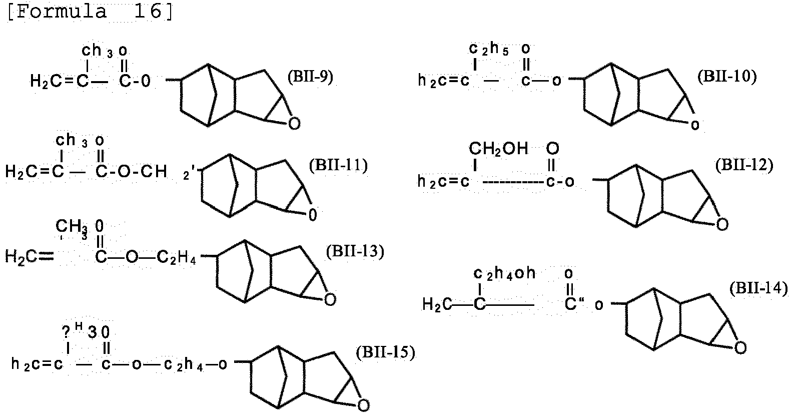

The compound represented by formula (BI) and the compound represented by formula (BII) may be used singly, or the compound represented by formula (BI) and the compound represented by formula (BII) may be used in combination. When these compounds are used in combination, the content rate between the compound represented by formula (BI) and the compound represented by formula (BII) is preferably 5:95 to 95:5, more preferably 10:90 to 90:10, and still more preferably 20:80 to 80:20, on a molar basis .
(b2) is more preferably a monomer having an oxetanyl group and a (meth) acryloyloxy group. Examples of (b2) include 3-methyl-3-methacryloyloxy methyl oxetane, 3-methyl-3-acryloyloxy methyl oxetane, 3-ethyl-3-methacryloyloxy methyl oxetane, 3-ethyl-3-acryloyloxy methyl oxetane, 3-methyl-3-methacryloyloxy ethyl oxetane, 3-methyl-3~acryloyloxy ethyl oxetane, 3-ethyl-3-methacryloyloxy ethyl oxetane, and 3-ethyl-3-acryloyloxy ethyl oxetane.
(b3) is more preferably a monomer having a tetrahydrofuryl group and a (meth) acryloyloxy group. Specific examples of (b3) include tetrahydrofurfuryl acrylate (for example, Viscoat V#150, manufactured by Osaka Organic Chemical Industry Ltd.) and tetrahydrofurfuryl methacrylate.
(b) is preferably (bl) since reliabilities such as heat resistance and Chemical resistance of a color filter to be produced can be further improved. Furthermore, (bl-2) is more preferable since the storage stability of the negative resist composition is excellent.

Examples of (c) include (meth)acrylic esters such as methyl (meth) acrylate, ethyl (meth) acrylate, n~ butyl(meth)acrylate, sec-butyl (meth)acrylate, tert-butyl (meth) acrylate, 2-ethylhexyl(meth) acrylate, dodecyl (meth) acrylate, lauryl (meth) acrylate, stearyl (meth) acrylate, cyclopentyl (meth) acrylate, cyclohexyl (meth) acrylate, 2-methylcyclohexyl(meth)acrylate, tricyclo[5.2.1.0^{2,6}]decan-8-yl (meth) acrylate (referred to as "dicyclopentanyl (meth) acrylate" (common name) in the art or sometimes referred to as "tricyclodecyl(meth)acrylate"), tricyclo [5.2.1.0^{2,6}]decene-8-yl (meth) acrylate (which is referred to as "dicyclopentenyl (meth) acrylate" (common name) in the art), dicyclopentanyloxyethyl (meth)acrylate, isobornyl (meth) acrylate, adamantyl (meth) acrylate, allyl (meth)acrylate, propargyl(meth) acrylate, phenyl (meth) acrylate, naphthyl (meth) acrylate, and benzyl (meth) acrylate;
hydroxy group-containing (meth)acrylic esters such as 2-hydroxyethyl(meth) acrylate and 2-hydroxypropyl (meth) acrylate;
dicarboxylic diesters such as diethyl maleate, diethyl fumarate, and diethyl itaconate;
bicyclo unsaturated compounds such as bicyclo[2.2.1]hept-2-ene, 5-methylbicyclo[2.2.1] hept-2-ene, 5-ethylbicyclo [2.2.1]hept-2-ene, 5-hydroxybicyclo [2.2.1]hept-2-ene, 5-hydroxymethylbicyclo [2.2.1]hept-2-ene, 5-(2'-hydroxyethyl) bicyclo[2.2.1] hept-2-ene, 5-methoxybicyclo[2.2.1] hept-2-ene, 5-ethoxybicyclo[2.2.1] hept-2-ene, 5,6-dihydroxybicyclo [2.2.1]hept-2-ene, 5,6-di(hydroxymethyl) bicyclo [2.2.1]hept-2-ene, 5,6-di (2'-hydroxyethyl) bicyclo[2.2.1]hept-2-ene, 5,6-dimethoxybi cyclo [2.2.1] hept-2-ene, 5,6-diethoxybicyclo[2.2.1] hept-2-ene, 5-hydroxy-5-methylbicyclo[2.2.1]hept-2-ene, 5-hydroxy-5-ethylbicyclo[2.2.1] hept-2-ene, 5-hydroxymethyl-5-methylbicyclo[2.2.1]hept-2-ene, 5-tert-but oxycarbony Ibicyclo [2.2.1]hept-2-ene, 5-cyclohexyloxycarbonylbicyclo[2.2.1] hept-2-ene, 5-phenoxycarbonylbicyclo[2.2.1]hept-2-ene, 5,6-bis(tert-butoxycarbonyl)bicyclo[2.2.1]hept-2-ene, and 5,6-bis (cyclohexyl oxycarbonyl) bicyclo [2.2.1]hept-2-ene;
dicarbonylimide derivatives such as N-phenylmaleimide, N-cyclohexylmaleimide, N-benzylmaleimide, N-succinimidyl-3-maleimidobenzoate, N-succinimidyl-4-maleimidobutyrate, N-succinimidyl-6-maleimide caproate, N-succinimidyl-3-maleimide propionate, and N- (9-acridinyl) maleimide;
styrene-based monomers such as styrene, **a-**methylstyrene, m-methylstyrene, p-methylstyrene, vinyltoluene, and p-methoxystyrene; nitrile-based monomers such as acrylonitrile and methacrylonitrile; halogenated vinyls such as vinyl Chloride and vinylidene Chloride; amide-based monomers such as acrylamide and methacrylamide; vinyl acetate; and diene-based monomers such as 1,3-butadiene, isoprene, and 2,3-dimethyl-l,3-butadiene .

Among these, from the viewpoint of copolymerization reactivity and heat resistance, styrene, vinyltoluene, N-phenylmaleimide, N-cyclohexylmaleimide, N-benzylmaleimide, bicyclo[2.2.1]hept-2-ene and the like are preferable.

In the resin [Kl], the ratio of the structural unit derived from each of (a) and (b) in the total structural units constituting the resin [Kl] is preferably the following:
the structural unit derived from (a); 2 to 60 mol%; and
the structural unit derived from (b); 40 to 98 mol%,
and more preferably the following:
   the structural unit derived from (a); 10 to 50 mol%; and
   the structural unit derived from (b); 50 to 90 mol%.

When the ratio of the structural unit of the resin [Kl] falls within the above-mentioned ränge, there is a tendency that the storage stability of the negative resist composition, that the developability thereof during the formation of a colored pattern, and that the solvent resistance of a color filter to be produced are excellent.

The resin [Kl] can be produced with reference to the method described in for example, a document "Experimental Method for Polymer Synthesis" (edited by Takayuki Otsu, published by Kagaku Dojin Publishing Co., Ltd., First Edition, First Printed on Mar. 1, 1972) and cited documents described in the above-mentioned document.

Specific examples thereof include the following method: predetermined amounts of (a) and (b), a polymerization Initiator, a solvent and the like are placed in a reaction vessel; for example, a deoxidization atmosphere is formed by substituting oxygen with nitrogen; and these are heated or kept warm during stirring. The polymerization Initiator, the solvent and the like which are used here are not particularly limited, and those commonly used in the art can be used. Examples of the polymerization Initiator include azo compounds (2,2'-azobisisobutyronitrile, 2,2'-azobis(2,4-dimethylvaleronitrile) and the like) and organic peroxides (benzoyl peroxide and the like). The solvent may be a solvent capable of dissolving each monomer, and examples of the solvent (B) for the negative resist composition of the present Invention include solvents to be described later.

A solution after a reaction, of the resultant Copolymer may be used as it is; a concentrated or diluted solution of the copolymer may be used; or a solid (powder) taken out from the copolymer by a method such as reprecipitation may be used. In particular, the solution after the reaction can be used as it is for preparing the negative resist composition of the present invention by using the solvent contained in the negative resist composition of the present invention as the solvent during the polymerization, whereby the producing process of the negative resist composition of the present invention can be simplified.

In the resin [K2] , the ratio of the structural unit derived from each of (a) to (c) in the total structural units constituting the resin [K2] is preferably the following :
the structural unit derived from (a); 2 to 45 mol%;
the structural unit derived from (b); 2 to 95 mol%; and
the structural unit derived from (c); 1 to 65 mol%,
and more preferably the following :
   the structural unit derived from (a); 5 to 40 mol%;
   the structural unit derived from (b); 5 to 80 mol%; and
   the structural unit derived from (c); 5 to 60 mol%.

When the ratio of the structural unit of the resin [K2] falls within the ränge, there is a tendency that the storage stability of the negative resist composition, the developability thereof during the formation of a colored pattern, and the solvent resistance, heat resistance, and mechanical strength of a color filter to be produced are excellent .

The resin [K2] can be produced in the same männer as in the method described as the producing method of the resin [Kl], for example.

In the resin [K3], the ratio of the structural unit derived from each of (a) and (c) in the total structural units constituting the resin [K3] is preferably the following:
the structural unit derived from (a); 2 to 60 mol%; and
the structural unit derived from (c); 40 to 98 mol%,
and more preferably the following:
   the structural unit derived from (a); 10 to 50 mol% and
   the structural unit derived from (c); 50 to 90 mol%.

The resin [K3] can be produced in the same männer as in the method described as the producing method of the resin [Kl], for example.

The resin [K4] can be produced by producing a copolymer of (a) and (c) and then adding a cyclic ether having 2 to 4 carbon atoms contained in (b) to a carboxylic acid and/or a carboxylic anhydride contained in (a).

The copolymer of (a) and (c) is first produced in the same männer as in the method described as the producing method of the resin [Kl]. In this case, the ratio of the structural unit derived from each of (a) and (c) is preferably the same ratio as that described in the resin [K3] .

Next, a cyclic ether having 2 to 4 carbon atoms contained in (b) is reacted with a part of the carboxylic acid and/or the carboxylic anhydride derived from (a) in the copolymer.

Subsequent to the production of the copolymer of (a) and (c)_{f} a nitrogen atmosphere in a flask is replaced with air, and (b), a reaction catalyst for a carboxylic acid or a carboxylic anhydride and a cyclic ether (for example, tris (dimethylaminomethyl) phenol and the like), and a polymerization inhibitor (for example, hydroquinone and the like) are placed in a flask, followed by reacting, for example, at 60 to 130°C for 1 to 10 hours, whereby the resin [K4] can be produced.

The amount of (b) used is preferably 5 to 80 mol, and more preferably 10 to 75 mol, relative to 100 mol of (a). The amount of (b) used falls within the above-mentioned ränge, whereby there is a tendency that the storage stability of the negative resist composition, the developability thereof during the formation of a pattern, and the balance of the solvent resistance, heat resistance, mechanical strength, and sensitivity of the pattern obtained are good. Since the reactivity of the cyclic ether is high, and the unreacted (b) is less likely to remain, (b) used for the resin [K4] is preferably (bl), and more preferably (bl-1).

The amount of the reaction catalyst used is preferably 0.001 to 5 parts by mass relative to 100 parts by mass of the total amount of (a), (b), and (c). The amount of the polymerization inhibitor used is preferably 0,001 to 5 parts by mass relative to 100 parts by mass of the total amount of (a), (b), and (c) .

Reaction conditions such as a feeding method, a reaction temperature, and time can be appropriately adjusted in consideration of a production eguipment, an amount of heat generation due to polymerization, and the like. In consideration of the production eguipment, the amount of heat generation due to polymerization, and the like, the feeding method and the reaction temperature can be appropriately adjusted like the polymerization conditions.

The resin [K5] is produced by producing a copolymer of (b) and (c) in the same männer as in the above-mentioned method for producing the resin [Kl] as a first step. In the same manner, a solution after a reaction, of the resultant copolymer may be used as it is; a concentrated or diluted solution of the copolymer may be used; or a solid (powder) taken out from the copolymer by a method such as reprecipitation may be used.

The ratio of the structural unit derived from each of (b) and (c) relative to the total number of moles of the total structural units constituting the copolymer is preferably the following:
the structural unit derived from (b) ; 5 to 95 mol%; and
the structural unit derived from (c); 5 to 95 mol%,
and more preferably the following:
   the structural unit derived from (b); 10 to 90 mol%; and
   the structural unit derived from (c); 10 to 90 mol%.

Furthermore, the resin [K5] can be produced by reacting a carboxylic acid or a carboxylic anhydride contained in (a) with the cyclic ether derived from (b) contained in the copolymer of (b) and (c) under the same conditions as those of the producing method of the resin [K4].

The amount of (a) used which is reacted with the copolymer is preferably 5 to 80 mol relative to 100 mol of (b). Since the reactivity of the cyclic ether is high, and the unreacted (b) is less likely to remain, (b) used for the resin [K5] is preferably (bl), and more preferably (bl-1).

The resin [K6] is a resin obtained by further reacting the resin [K5] with a carboxylic anhydride.

A carboxylic anhydride is reacted with a hydroxy group generated by a reaction between a cyclic ether and a carboxylic acid or a carboxylic anhydride.

Examples of the carboxylic anhydride include succinic anhydride, maleic anhydride, citraconic anhydride, itaconic anhydride, 3-vinylphthalic anhydride, 4-vinylphthalic anhydride, 3,4,5,6-tetrahydrophthalic anhydride, 1,2,3,6-tetrahydrophthalic anhydride, dimethyltetrahydrophthalic anhydride, and 5,6-dicarboxybicyclo[2.2.1]hept-2-ene anhydride (himic anhydride) . The amount of the carboxylic anhydride used is preferably 0.5 to 1 mol relative to 1 mol of the amount of (a) used.

Specific examples of the resin (C) include a resin [Kl] such as a 3,4-epoxycyclohexylmethyl(meth)acrylate/(meth)acrylic acid copolymer or a 3,4-epoxytricyclo[5.2.1.0^{2,6}]decyl acrylate/ (meth) acrylic acid copolymer; a resin [K2] such as a
glycidyl (meth) acrylate/benzyl(meth)acrylate/ (meth) acrylic acid copolymer, a
glycidyl(meth) acrylate/styrene/(meth)acrylic acid copolymer, a 3,4-epoxytricyclo[5.2.1.0^{2,6}]decyl acrylate/ (meth)acrylic acid/N-cyclohexylmaleimide copolymer, 3,4-epoxytricyclo [5.2.1.0^{2,6}]decyl acrylate/ (meth) acrylic acid/styrene-based monomer Copolymer, or a 3-methyl-3-(meth)acryloyloxymethyl oxetane/(meth)acrylic acid/styrene Copolymer; a resin [K3] such as a benzyl (meth) acrylate/ (meth) acrylic acid copolymer or a styrene/(meth) acrylic acid copolymer; a resin [K4] such as a resin produced by adding glycidyl(meth) acrylate to a
benzyl (meth) acrylate/ (meth) acrylic acid copolymer, a resin produced by adding glycidyl (meth) acrylate to a tricyclodecyl(meth)acrylate/styrene/(meth)acrylic acid copolymer, or a resin produced by adding glycidyl (meth) acrylate to a
tricyclodecyl (meth) acrylate/benzyl (meth)acrylate/ (meth) ac rylic acid copolymer; a resin [K5] such as a resin produced by reacting a
tricyclodecyl (meth) acrylate/glycidyl (meth) acrylate copolymer with (meth) acrylic acid or a resin produced by reacting a
tricyclodecyl (meth) acrylate/styrene/glycidyl(meth) acrylat e copolymer with (meth) acrylic acid; and a resin [K6] such as a resin produced by reacting a tricyclodecyl (meth)acrylate/glycidyl(meth)acrylate copolymer with (meth) acrylic acid to produce a resin and then reacting this resin with tetrahydrophthalic anhydride .

Among these, the resin (C) is preferably the resin [Kl] and/or the resin [K2].

The weight average molecular weight of the resin (C) in terms of polystyrene is preferably 3,000 to 100,000, more preferably 5,000 to 50,000, and still more preferably 5,000 to 30,000. The molecular weight distribution [weight average molecular weight (Mw) /number average molecular weight (Mn)] of the resin (C) is preferably 1.1 to 6, and more preferably 1.2 to 4.

The acid value of the resin (C) is preferably 50 to 170 mg KOH/g, more preferably 60 to 150 mg KOH/g, and still more preferably 70 to 135 mg KOH/g. The acid value as used herein is a value which is measured as an amount (mg) of potassium hydroxide required for neutralizing 1 g of the resin, and which can be determined by, for example, titration with an aqueous potassium hydroxide solution .

### <Dispersant (D) >

The negative resist composition of the present invention preferably further contains a dispersant (D) . The dispersant (D) is not particularly limited as long as it is used for dispersing a colorant, and has an amine group and an amine value of 0 to 55 mg KOH/g (preferably 2 to 40 mg KOH/g), and.examples thereof include a polymeric dispersant.

Examples of the polymeric dispersant include an acrylic dispersant and a urethane dispersant.

Examples of the acrylic dispersant include an acrylic block copolymer, and it is preferred to use, as the acrylic block copolymer, a block copolymer having a colorant adsorbing block containing a basic group as a colorant adsorbing group (also referred to as dye adsorbing group) and also containing an acidic group as a colorant adsorbing group, and a block without a colorant adsorbing group.

Examples of the colorant adsorbing block containing a basic group and also containing an acidic group as colorant adsorbing groups include a block constituted by using a monomer having a basic group in combination with a monomer having an acidic group.

Examples of the monomer having a basic group include a monomer having a primary amino group, a secondary amino group, a tertiary amino group, or a quaternary ammonium group .

Specific examples include N,N-dimethylaminoethyl (meth)acrylate, N,N-diethylaminoethyl (meth) acrylate, N,N-dimethylacrylamide, diethylacrylamide, dimethylaminopropyl methacrylamide, acryloylmorpholine, vinylimidazole, 2-vinylpyridine, a monomer having an amino group and a caprolactone skeleton, a reaction product of a monomer having a glycidyl group such as glycidyl (meth)acrylate and a compound having a single secondary amino group in its molecule, and a reaction product of a (meth) acryloylalkyl isocyanate compound with 4-(2-aminomethyl) -pyridine, 4-(2-aminoethyl)-pyridine, 4-(2-hydroxyethyl) pyridine, 1- (2-aminoethyl) -piperazine, 2-amino-6-methoxybenzothiazole, 1-(2-hydroxyethylimidazole), N,N-diarylmelamine, or N,N-dimethyl-1 ,3-propanediamine.

Examples of the monomer having an acidic group include a monomer having a carboxy group, a sulfonic acid group, or a phosphate group. Specific examples of the monomer having a carboxy group include an unsaturated monocarboxylic acid compound such as acrylic acid, methacrylic acid, or crotonic acid, an unsaturated dicarboxylic acid compound such as maleic acid, fumaric acid, or itaconic acid, and a half ester thereof; specific examples of the monomer having a sulfonic acid group include 2-acrylamido-2-methyl-1-propanesulfonic acid, 2-methacrylamido-2-methyl-1-propanesulfonic acid, and styrenesulfonic acid; and specific examples of the monomer having a phosphate group include acidic phosphonyl (meth)acrylate and acidic phosphonyl ethyl (meth)acrylate.

Examples of a constituent of the block without a colorant adsorbing group include an aromatic vinyl compound such as styrene, a-methylstyrene, vinyl toluene, or benzyl Chloride, an unsaturated carboxylic acid alkyl ester such as methyl (meth) acrylate, ethyl (meth) acrylate, or butyl (meth) acrylate, an unsaturated carboxylic acid aryl alkyl ester such as benzyl (meth)acrylate, a polycaprolactone-containing monomer, and a polyalkylene glycol monoester-based monomer. The acrylic block copolymer can be obtained by living anionic polymerization or the like, and a conventionally known polymerization method can be used.

The amine value of the acrylic block copolymer is 0 to 55 mg KOH/g, preferably 0 to 50 mg KOH/g, and more preferably 2 to 40 mg KOH/g. The amine value means an amine value per g of solids in the acrylic block copolymer; the value is measured by potentiometric titration (for example, COMTITE (AUTOTITRATOR COM-900, BURET B-900, TITSTATIONK-900 ), manufactured by Hiranuma Sangyo Co. Ltd.) using a 0.1 mol/L aqueous solution of hydrochloric acid, and then converted into potassium hydroxide equivalents.

Examples of commercially available products of the acrylic block copolymer include "Disperbyk (registered trademark)-112 (amine value: 36 mg KOH/g)", "Disperbyk (registered trademark)-2000 (amine value: 4 mg KOH/g)", "Disperbyk-2001 (amine value: 29 mg KOH/g)", "Disperbyk (registered trademark)-2020 (amine value: 38 mg KOH/g)", "Disperbyk (registered trademark)-2050 (amine value: 30 mg KOH/g)", and "Disperbyk (registered trademark) -2070 (amine value: 20 mg KOH/g)" manufactured by BYK-Chemie Japan.

As the urethane dispersant, a urethane dispersant can be used that is obtained by reacting isocyanate groups of a polyisocyanate compound with a compound having one or more hydroxy groups in its molecule and having a number average molecular weight of 300 to 10,000 and a basic group-containing compound having a functional group that can react with an isocyanate group in its molecule. The method disclosed in Japanese Patent Laid-Open No. 60-166318 or the like can be used as a method for obtaining such a urethane dispersant.

Examples of the polyisocyanate compound constituting the urethane dispersant include an isocyanate compound having two or more isocyanate groups, for example, an aromatic diisocyanate compound such as 2,4-tolylenediisocyanate, a dimer of 2,4-tolylenediisocyanate, 2,6-tolylenediisocyanate, p-xylene diisocyanate, m-xylene diisocyanate, 4,4'-diphenylmethane diisocyanate, 1,5-naphthylene diisocyanate, or 3,3'-dimethylbiphenyl-4 ,4'-diisocyanate; an aliphatic or alicyclic polyisocyanate such as hexamethylene diisocyanate, isophorone diisocyanate, 4,4'-methylenebis(cyclohexyl isocyanate), methylcyclohexane-2,4(or 2,6)diisocyanate, or 1,3-(isocyanatomethylene) cyclohexane; a polyisocyanate having an isocyanuric group based on the above-mentioned diisocyanate (such as a polyisocyanate having an isocyanuric group formed by trimerization of the above-mentioned diisocyanate), a polyisocyanate obtained by reacting a polyol with a diisocyanate, and a polyisocyanate obtained by biuret reaction of a diisocyanate compound. Among the above-mentioned polyisocyanate Compounds, for example, a polyisocyanate having an isocyanuric group based on a diisocyanate such as tolylene diisocyanate or isophorone diisocyanate is preferred.

Examples of the compound having one or more hydroxy groups in its molecule constituting the urethane dispersant include a polyether compound and a polyester compound.

Examples of the polyether compound include polyalkylene glycols such as polyethylene glycol, Polypropylens glycol, polybutylene glycol, and polytetramethylene glycol; and alkylene glycols such as ethylene glycol, propanediol, propylene glycol, tetramethylene glycol, pentamethylene glycol, hexanediol, neopentyl glycol, glycerin, trimethylolpropane , pentaerythritol, diglycerin, ditrimethylolpropane, and dipentaerythritol, and low molecular weight monols such as methanol and ethanol, modified with ethylene oxide, propylene oxide, butylene oxide, tetrahydrofuran and the like.

Examples of the polyester compound include alkylene glycols such as ethylene glycol, propanediol, propylene glycol, tetramethylene glycol, pentamethylene glycol, hexanediol, neopentyl glycol, glycerin, trimethylolpropane, pentaerythritol, diglycerin, ditrimethylolpropane, and dipentaerythritol, and low molecular weight monols such as methanol and ethanol, modified with e-caprolactone, y-butyrolactone, ö-valerolactone, and methyl valerolactone; an aliphatic polyester polyol that is an esterification product of an aliphatic dicarboxylic acid such as adipic acid or dimer acid with a polyol such as neopentyl glycol or methylpentanediol; a polyester polyol such as an aromatic polyester polyol that is an esterification product of an aromatic dicarboxylic acid such as terephthalic acid with a polyol such as neopentyl glycol; an esterification product of a polyhydric hydroxy group compound such as a polycarbonate polyol, an acrylic polyol, or polytetramethylene hexaglyceryl ether (hexaglycerin modified with tetrahydrofuran) with a dicarboxylic acid such as fumaric acid, phthalic acid, isophthalic acid, itaconic acid, adipic acid, sebacic acid, or maleic acid; and a polyhydric hydroxy group-containing compound such as a monoglyceride obtained by ester-exchange reaction of a polyhydric hydroxy group-containing compound such as glycerin with a fatty acid ester. Among the above-mentioned compounds having one or more hydroxy groups in its molecule, an £-caprolactone adduct of an alcohol is preferred.

The number average molecular weight of the compound having one or more hydroxy groups in its molecule is 300 to 10,000, and preferably 300 to 6,000. The number average molecular weight and the weight average molecular weight can be measured by column chromatography .

The basic group-containing compound having a functional group that can react with an isocyanate group in its molecule constituting the urethane dispersant is not particularly limited, and is preferably at least one compound selected from the group consisting of a polyol, a polythiol, and an amine having an N,N-disubstituted amino group or a heterocyclic nitrogen atom. Compounds known and conventionally used in the art of dispersants can be used as these compounds. These compounds have a zerewitinoff-active hydrogen atom and at least one nitrogen atom-containing basic group. Examples of such compounds include N,N-dimethyl-1 ,3-propanediamine, N,N-diethyl-1,4-butanediamine, 2-dimethylaminoethanol, 1-(2-aminoethyl) -piperazine, 2-(1-pyrrolidyl) -ethylamine, 4-amino-2-methoxypyrimidine, 4-(2-aminoethyl )-pyridine, 1-(2-hydroxyethyl) -piperazine, 4-(2-hydroxyethyl)-morpholine, 2-mercaptopyrimidine, *2-*mercaptobenzimidazole, 2-amino-6-methoxybenzothiazole, N,N-diallyl-melamine, 3-amino-1,2,4-triazole, l-(2-hydroxyethyl)-imidazole, and 3-mercapto-l, 2,4-triazole . Among these, an amine having a heterocyclic nitrogen atom is preferred.

The reaction to synthesize the urethane dispersant is not particularly limited, and can be carried out by a conventionally known method. The amine value of the urethane dispersant is also 0 to 55 mg KOH/g, and preferably 5 to 40 mg KOH/g.

Examples of commercially available products of the urethane dispersant include Disperbyk-161 (amine value: 11 mg KOH/g, manufactured by BYK-Chemie), Disperbyk-1 62 (amine value: 13 mg KOH/g manufactured by BYK-Chemie), Disperbyk-167 (amine value: 13 mg KOH/g, manufactured by BYK-Chemie) , and Disperbyk-182 (amine value: 13 mg KOH/g, manufactured by BYK-Chemie).

The acrylic dispersant is preferred as the dispersant .

The negative resist composition of the present invention preferably further contains a polymerizable compound (E) and a polymerization Initiator (F). This allows the negative resist composition to exhibit curability during exposure or the like.

### <Polymerizable Compound (E)>

The polymerizable compound (E) is a compound capable of being polymerized by the action of an active radical, an acid or the like generated from the polymerization Initiator (F) to be described later. Examples of the polymerizable compound (E) include a compound having a polymerizable ethylenically unsaturated bond, and is preferably a (meth) acrylic acid ester compound, for example.

Among these, the polymerizable compound (E) is preferably a polymerizable compound having three or more (preferably 4 to 10, more preferably 5 to 8) ethylenically unsaturated bonds, and more preferably an ester of an alcohol having 3 or more (preferably 4 to 10, more preferably 5 to 8) OH groups (for example, pentaerythritol, a condensation product thereof, or a modified product thereof) with (meth) acrylic acid. Examples of the polymerizable compound include pentaerythritol tetra(meth)acrylate, dipentaerythritol penta(meth) acrylate, dipentaerythritol hexa (meth) acrylate, tripentaerythritol octa (meth) acrylate, tripentaerythritol hepta (meth)acrylate, tetrapentaerythritol deca(meth) acrylate, tetrapentaerythritol nona (meth) acrylate, tris (2-(meth)acryloyloxyethyl)isocyanurate, ethylene glycol-modified pentaerythritol tetra(meth) acrylate, ethylene glycol-modified dipentaerythritol hexa (meth) acrylate, propylene glycol-modified pentaerythritol tetra (meth) acrylate, propylene glycol-modif ied dipentaerythritol hexa(meth) acrylate, caprolactone-modified pentaerythritol tetra(meth) acrylate, and caprolactone-modified dipentaerythritol hexa (meth) acrylate . Among these, preferred are dipentaerythritol penta(meth) acrylate and dipentaerythritol hexa (meth) acrylate, for example.

The weight average molecular weight of the polymerizable compound (E) is preferably 150 or more and 2,900 or less, and more preferably 250 or more and 1,500 or less.

The content of the polymerizable compound (E) is preferably 7 to 65% by mass, more preferably 13 to 60% by mass, and still more preferably 17 to 55% by mass, relative to the solid content of the negative resist composition. The content of the polymerizable compound (E) preferably falls within the above-mentioned ränge, because there is a tendency that the curing occurs sufficiently, the residual film ratio in development is improved, and an undercut is less likely to form in the colored pattern to provide good adhesion.

### <Polymerization Initiator (F)>

The polymerization Initiator (F) is not particularly limited, as long as the polymerization Initiator (F) is a compound capable of generating active radicals, an acid or the like by the action of light or heat to initiate polymerization. Any known polymerization Initiator can be used.

A compound that generates active radicals by the action of light is preferred as the polymerization Initiator (F), and an alkylphenone compound, a triazine compound, an acylphosphine oxide compound, an oxime compound, and a biimidazole compound are more preferred.

The alkylphenone compound is a compound having a partial structure represented by formula (d2) or a partial structure represented by formula (d3). In these partial structures, the benzene ring optionally has a substituent. In the formula, * represents a point of attachment.

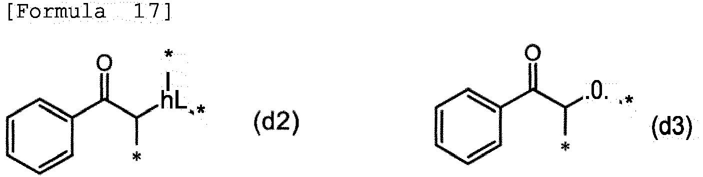

Examples of the compound having a partial structure represented by formula (d2) include 2-methyl~2-morpholino-1-(4-methylsulfanylphenyl)propane-l-one, 2-dimethylamino-l-(4-morpholinophenyl )-2-benzylbutane-l-one, and 2-(dimethylamino)-2-[(4-methylphenyl) methyl]-1-[4-(4-morpholinyl)phenyl]butane-l-one. Commercially available products such as Irgacure (registered trademark) 369, 907, and 379 (all manufactured by BASF Corporation) may be used.

Examples of the compound having a partial structure represented by formula (d3) include 2-hydroxy-2-methyl-l-phenylpropane-l-one, 2-hydroxy-2-methyl-l- [4- (2-hydroxyethoxy)phenyl]propane-l-one, 1-hydroxycyclohexylphenylketone, an oligomer of 2-hydroxy-2-methyl-l- (4-isopropenylphenyl)propane-l-one, a,a-diethoxyacetophenone, and benzyl dimethyl ketal.

The alkylphenone compound is preferably a compound having a partial structure represented by formula (d2) from the viewpoint of sensitivity, and more preferably 2-methyl-2-morpholino-1-(4-methylsulfanylphenyl) propan-1-one or 2-dimethylamino-1-(4-morpholinophenyl) -2-benzylbutan-l-one.

Examples of the triazine compound include 2,4-bis(trichloromethyl )-6-(4-methoxyphenyl) -1,3,5-triazine, 2,4-bis(trichloromethyl)-6-(4~methoxynaphthyl)-1,3,5-triazine, 2,4-bis (trichloromethyl)-6-piperonyl-l ,3,5-triazine, 2,4-bis(trichloromethyl) -6-(4-methoxystyryl )-1.3.5-triazine, 2,4-bis (trichloromethyl) -6-[2-(5-methylfuran-2-yl)ethenyl]-1,3,5-triazine, 2,4-bis(trichloromethyl)-6-[2-(furan-2-yl) ethenyl]-1,3,5-triazine, 2,4-bis(trichloromethyl) -6-[2- (4-diethylamino-2-methylphenyl) ethenyl] -1,3,5-triazine, and 2,4-bis(trichloromethyl)-6-[2-(3,4-dimethoxyphenyl)ethenyl]-1.3.5-triazine.

Examples of the acylphosphine oxide compound include 2.4.6-trimethylbenzoyldiphenylphosphine oxide. Commercially available products such as Irgacure (registered trademark) 819 (manufactured by BASF Corporation) may be used.

The oxime compound is a compound having a partial structure represented by formula (dl). Hereinafter, * represents a point of attachment.

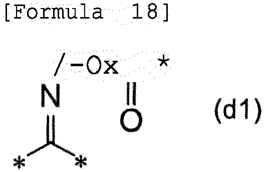

Examples of the oxime compound include N-benzoyloxy-1-(4-phenylsulfanylphenyl )butan-l-one-2-imine, N-benzoyloxy-1- (4-phenylsulf anylphenyl) octan-l-one-2-imine, N-benzoyloxy-1- (4-phenylsulfanylphenyl) -3-cyclopentylpropan-l-one-2-imine, N-acetoxy-1-[9-ethyl-6-(2-methylbenzoyl )-9H-carbazol-3-yl ]ethane-l-imine, N-acetoxy-1- [9-ethyl-6-{2-methyl-4-(3,3-dimethyl-2 ,4-dioxacyclopentanylmethyloxy)benzoyl}-9H-carbazol-3-yl]ethane-l-imine, N-acetoxy-1- [9-ethyl-6-(2-methylbenzoyl)-9H-carbazol-3-yl]-3-cyclopentylpropane-l-imine, and N-benzoyloxy-1- [9-ethyl-6- (2-methylbenzoyl)-9H-carbazol-3-yl] -3-cyclopentylpropan-l-one-2-imine. Commercially available products such as Irgacures (registered trademark) OXEOl and OXE02 (all manufactured by BASF Corporation), and N-1919 (manufactured by ADEKA Corporation) may be used. Among these, preferred are N-benzoyloxy-1- (4-phenylsulfanylphenyl) butan-l-one- 2-imine, N-benzoyloxy-1- (4-phenylsulfanylphenyl)octan-l-one-2-imine, and N-benzoyloxy-1- (4-phenylsulfanylphenyl)-3-cyclopentylpropan-l-one-2-imine. There is a tendency that these oxime compounds provide a color filter having a high brightness when the negative resist composition of the present invention is prepared as a blue color negative resist composition.

Examples of the biimidazole compound include 2,2'-bis(2-chlorophenyl)-4,4',5,5'-tetraphenylbiimidazole, 2,2'-bis(2,3-dichlorophenyl)-4,4',5,5'-tetraphenylbiimidazole (for example, see Japanese Patent Laid-Open No. 6-75372 and Japanese Patent Laid-Open No. 6-75373), 2,2'-bis(2-chlorophenyl ) -4,4',5,5'-tetraphenyl biimidazole, 2,2'-bis(2-chlorophenyl) -4,4',5,5'-tetra(alkoxyphenyl )biimidazole, 2,2'-bis(2-chlorophenyl )-4,4',5,5'-tetra (dialkoxyphenyl )biimidazole, 2,2'-bis(2-chlorophenyl) -4,4',5,5'-tetra (trialkoxyphenyl) biimidazole (for example, see Japanese Patent No. 48-38403 and Japanese Patent Laid-Open No. 62-174204), and an imidazole compound in which a phenyl group at the 4,4',5,5' position is substituted with a carboalkoxy group (for example, see Japanese Patent Laid-Open No. 7-10913). Preferred are, for example, 2,2'-bis(2-chlorophenyl) -4,4',5,5'-tetraphenylbiimidazole, 2,2'-bis (2, 3-dichlorophenyl) -4,4',5, 5'-tetraphenylbiimidazole, and 2,2'-bis(2,4-dichlorophenyl)-4,4'5,5'-tetraphenylbiimidazole.

Furthermore, examples of the polymerization Initiator (F) include benzoin compounds such as benzoin, benzoin methyl ether, benzoin ethyl ether, benzoin isopropyl ether, and benzoin isobutyl ether; benzophenone compounds such as benzophenone, methyl o-benzoylbenzoate, 4-phenylbenzophenonne, 4-benzoyl-4 '-methyldiphenyl sulfide, 3,3',4,4'-tetra(tert-butylperoxycarbonyl)benzophenone, and 2,4,6-trimethylbenzophenone; quinone compounds such as 9, 10-phenanthrene quinone, 2-ethylanthraquinone, and camphorquinone; 10-butyl-2-chloroacridone, benzyl, methyl phenylglyoxylate, and a titanocene compound. These are preferably used in combination with a polymerization Initiation aid (Fl) (particularly, amines) to be described later.

Examples of an acid generator that generates an acid by the action of light include onium salts such as 4-hydroxyphenyldimethylsulfonium p-toluenesulfonate, 4-hydroxyphenyldimethylsulfoniumhexaf luoroantimonate, 4-acetoxyphenyldimethylsulf onium p-toluenesulfonate, 4-acetoxyphenyl-methyl -benzylsulfoniumhexafluoroantimonate, triphenylsulfonium p-toluenesulfonate, triphenylsulfonium hexafluoroantimonate, diphenyliodonium p-toluenesulf onate, and diphenyliodoniumhexafluoroantimonate, nitrobenzyl tosylates, and benzoin tosylates.

The content of the polymerization Initiator (F) is preferably 0.1 to 30 parts by mass, and more preferably 1 to 20 parts by mass, relative to 100 parts by mass of the whole amount of the resin (C) (including the resin (C') to be described later, if it is included) and the polymerizable compound (E) in the negative resist composition of the present invention. When the content of the photopolymerization Initiator falls within the above-mentioned ränge, the sensitivity is increased, and the time of exposure to light is shortened, resulting in the improvement in productivity .

The negative resist composition of the present invention may further contain at least one selected from the group consisting of a polymerization Initiation aid (Fl) and a leveling agent (G).

### <Polymerization Initiation Aid (Fl)>

The negative resist composition of the present invention may further contain the polymerization Initiation aid (Fl). The polymerization Initiation aid (Fl) is a compound to be used for accelerating polymerization of a polymerizable compound the polymerization of which has been started by the polymerization Initiator, or a sensitizer. The polymerization Initiation aid (Fl) is usually used in combination with the polymerization Initiator (F) .

Examples of the polymerization Initiation aid (Fl) include an amine compound, an alkoxyanthracene compound, a thioxanthone compound, and a carboxylic acid compound.

Examples of the amine compound include triethanolamine, methyldiethanolamine, triisopropanolamine, methyl 4-dimethylaminobenzoate, ethyl 4-dimethylaminobenzoate, isoamyl 4-dimethylaminobenzoate, 2-dimethylaminoethyl benzoate, 2-ethylhexyl 4-dimethylaminobenzoate, N,N-dimethylparatoluidine, 4,4'-bis(dimethylamino)benzophenone (common name: Michler's ketone), 4,4'-bis (diethylamino) benzophenone, and 4,4'-bis (ethylmethylamino) benzophenone. Among these, 4,4'-bis (diethylamino)benzophenone is preferred. Commercially available products such as EAB-F (manuf actured by Hodogaya Chemical Co., Ltd.), may be used.

Examples of the alkoxyanthracene compound include 9,10-dimethoxyanthracene, 2-ethyl-9, 10-dimethoxyanthracene, 9,10-diethoxyanthracene, 2-ethyl-9,10-diethoxyanth.racene, 9,10-dibutoxyanthracene, and 2-ethyl-9, 10-dibutoxyanthracene.

Examples of the thioxanthone compound include 2-isopropylthioxanthone, 4-isopropylthioxanthone, 2,4-diethylthioxanthone, 2,4-dichlorothioxanthone, and 1-chloro-4-propoxythioxanthone .

Examples of the carboxylic acid compound include phenylsulfanylacetic acid, methylphenylsulf anylacetic acid, ethylphenylsulfanylacetic acid, methylethylphenylsulfanylacetic acid, dimethylphenylsulfanylacetic acid, methoxyphenysulf anylacetic acid, dimethoxyphenylsulf anylacetic acid, chlorophenylsulf anylacetic acid, dichlorophenylsulf anylacetic acid, N-phenylglycine, phenoxyacetic acid, naphthylthioacetic acid, N-naphthylglycine, and naphthoxyacetic acid.

When the polymerization Initiation aid (Fl) is used, the amount of the polymerization Initiation aid (Fl) used is preferably 0.1 to 30 parts by mass, and more preferably 1 to 20 parts by mass, relative to 100 parts by mass of the whole amount of the resin (C) (including the resin (C') to be described later, if it is included) and the polymerizable compound (E) in the negative resist composition of the present invention. When the amount of the polymerization Initiation aid (Fl) falls within the above-mentioned ränge, there is a tendency that the pattern can be formed with higher sensitivity, resulting in the improvement in productivity of the pattern.

### <Leveling Agent (G)>

Examples of the leveling agent (G) include a silicone-based Surfactant, a fluorine-based Surfactant, and a silicone-based Surfactant having a fluorine atom. These may have a polymerizable group at its side chain.

Examples of the silicone-based surfactant include a Surfactant having a siloxane bond. Specific examples thereof include Toray Silicone (trade name) DC3PA, Toray Silicone SH7PA, Toray Silicone DC11PA, Toray Silicone SH21PA, Toray Silicone SH28PA, Toray Silicone SH29PA, Toray Silicone SH30PA, and Toray Silicone SH8400 (manufactured by Dow Corning Toray Co., Ltd.); KP321, KP322, KP323, KP324, KP326, KP340, and KP341 (manufactured by Shin-Etsu Chemical Co., Ltd.); and TSF400, TSF401, TSF410, TSF4300, TSF4440, TSF4445, TSF-4446, TSF4452, and TSF4460 (manuf actured by Momentive Performance Materials Inc.).

Examples of the fluorine-based surfactant include a surfactant having a fluorocarbon chain. Specific examples thereof include Fluorad (registered trademark) FC430 and Fluorad FC431 (manufactured by Sumitomo 3M, Ltd.); Megafac (registered trademark) F142D, Megafac F171, Megafac F172, Megafac F173, Megafac F177, Megafac F183, Megafac F554, Megafac R30, and Megafac RS-718-K (manufactured by DIC Corporation) ; Eftop (registered trademark) EF301, Eftop EF303, Eftop EF351, and Eftop EF352 (manufactured by Mitsubishi Materials Electronic Chemicals Co., Ltd.); Surflon (registered trademark) S381, Surflon S382, Surflon SC101, and Surflon SC105 (manufactured by Asahi Glass Co., Ltd.); and E5844 (manufactured by Daikin Finechemical Laboratory).

Examples of the silicone-based Surfactant having a fluorine atom include a Surfactant having a siloxane bond and a fluorocarbon chain. Specific examples thereof include Megafac (registered trademark) R08, Megafac BL20, Megafac F475, Megafac F477, and Megafac F443 (manufactured by DIC Corporation) .

The content of the leveling agent (G) is preferably 0.001% by mass or more and 0.2% by mass or less, preferably 0.002% by mass or more and 0.1% by mass or less, and more preferably 0.005% by mass or more and 0.05% mass or less, relative to the whole amount of the negative resist composition of the present invention. When the content of the leveling agent (G) falls within the above-mentioned ränge, the color filter can be provided with good flatness.

### <Other Components>

If required, the negative resist composition of the present invention may contain various additives known in the art (hereinafter referred to as "other components"), such as a filier, other polymeric compound, an adhesion Promoter, an antioxidant, a light stabilizer, and a chain transfer agent.

### <Method for Producing Negative Resist Composition>

The negative resist composition can be obtained by, for example, subjecting the colorant (A) and the resin (C) to a dispersion treatment in the solvent (B) containing the dispersant (D) to obtain a colorant dispersion, and then, if required, mixing the polymerizable compound (E), the polymerization Initiator (F), the leveling agent (G) and the like.

The dispersion treatment means mixing particles such as the colorant (A) and the resin (C) until a dispersion state is achieved. The particles are ground to smaller particles by this dispersion treatment. The dispersion state means a state in which the particles float in the solvent (B) in the mixture.

The content of the colorant (A) in the colorant dispersion is preferably 2% by mass or more, and more preferably 5% by mass or more, while it is preferably 30% by mass or less, and more preferably 20% by mass or less, relative to the whole amount of the colorant dispersion.

The content of the solvent (B) is preferably 60% by mass or more, and more preferably 75% by mass or more, while it is preferably 93% by mass or less, more preferably 90% by mass or less, and most preferably 85% by mass or less, relative to the whole amount of the colorant dispersion.

When the colorant dispersion contains the resin (C), the content of the resin (C) is preferably 1% by mass or more, and more preferably 2% by mass or more, while it is preferably 15% by mass or less, and more preferably 7% by mass or less, relative to the whole amount of the colorant dispersion. When the content of the resin (C) falls within the above-mentioned ränge, there is a tendency that the dispersion state is stable.

The content of the dispersant (D) in the colorant dispersion is preferably 1% by mass or more, and more preferably 2% by mass or less, while it is preferably 20% by mass or less, and more preferably 10% by mass or less, relative to the whole amount of the colorant dispersion. When the content of the dispersant (D) falls within the above-mentioned ränge, there is a tendency that the dispersion state is stable.

The temperature for dispersing the colorant (A) in the solvent (B) and for dispersing the mixture is preferably 120°C or lower, and more preferably 70°C or lower. The lower limit of the temperature for dispersing is not particularly limited, and is usually 20°C. The dispersing time is preferably 0.5 hour or more, and preferably 2 hours or more, while it is preferably 48 hours or less, and more preferably 20 hours or less. Examples of devices to be used for dispersing include a roll mill, a high-speed stirrer, a bead mill, a ball mill, a sand mill, a paint conditioner, an ultrasonic dispersion machine, and a high-pressure dispersion machine. The resultant colorant dispersion is preferably filtered through a filter with a pore size of about 1.0 to 5.0 |im.

When the polymerizable compound (E) and the polymerization Initiator (F) are added to the colorant dispersion, it is preferred to further add a resin ("resin (C')"). Examples of the resin (C') include the same as those of the resin (C). The resin (C*) may be the same as or different in type from the resin (C). Preferred as the resin (C) are the resin [Kl], resin [K2], resin [K5], and resin [K6], more preferred are the resin [K5] and resin [K6], and still more preferred is the resin [K6] .

The total content of the resin (C) and the resin (C') is preferably 7 to 65% by mass, more preferably 10 to 60% by mass, still more preferably 13 to 60% by mass, and particularly preferably 13 to 55% by mass, relative to the whole amount of the solid content. When the content of the resins falls within the above-mentioned ränge, there is a tendency that the resolution of the colored pattern and the residual film ratio of the colored pattern are improved.

When the polymerizable compound (E) and the polymerization Initiator (F) are added to the colorant dispersion, it is preferred to further add a solvent (B').

Examples of the solvent (B') include the same as those of the solvent (B) . The solvent (B') is preferably an organic solvent having a boiling point of 120°C or higher and 180°C or lower at 1 atm from the viewpoint of applicability and drying performance, and more preferably propylene glycol monomethyl ether acetate, ethyl lactate, propylene glycol monomethyl ether, ethyl 3-ethoxypropionate, ethylene glycol monomethyl ether, diethylene glycol monomethyl ether, diethylene glycol monoethyl ether, diethylene glycol ethyl methyl ether, 3-methoxybutyl acetate, 3-methoxy-l-butanol, 4-hydroxy-4-methyl-2-pentanone, N,N-dimethylformamide or the like, and still more preferably propylene glycol monomethyl ether acetate, ethyl lactate, 4-hydroxy-4-methyl-2-pentanone, diethylene glycol ethyl methyl ether, 3-methoxybutyl acetate, 3-methoxy-l-butanol, ethyl 3-ethoxypropionate or the like.

The total content of the solvent (B) and the solvent (B') in the negative resist composition is preferably 40 to 95% by mass, and more preferably 45 to 92% by mass, relative to the whole amount of the negative resist composition. In other words, the solid content of the negative resist composition is preferably 5 to 60% by mass, and more preferably 8 to 55% by mass. When the content of the solvents falls within the above-mentioned ränge, there is a tendency that the flatness during application becomes good and the color density of the color filter formed becomes not insufficient, resulting in the achievement of good displaying properties.

Examples of the method for producing the colored pattern of the color filter from the negative resist composition of the present invention include a photolithography method, an inkjet method, and a printing method. Among these, a photolithography method is preferable. The photolithography method is a method in which the negative resist composition is applied onto a Substrate and then dried to form a composition layer, and the composition layer is then developed by exposing the composition layer to light through a photomask. In the photolithography method, the photomask is not used during the exposure to light, and/or the composition layer is not developed, whereby a colored coating film which is a cured product of the composition layer can be formed.

The film thickness of the color filter (cured film) is preferably 20 pm or less, more preferably 6 pm or less, still more preferably 3 pm or less, even more preferably 1.5 pm or less, and particularly preferably 0.5 pm or less, while it is preferably 0.1 pm or more, more preferably 0.2 pm or more, and still more preferably 0.3 pm or more.

Examples of the Substrate to be used include glass Substrates such as quartz glass, borosilicate glass, alumina silicate glass, and soda lime glass of which the surface is coated with silica; resin Substrates such as polycarbonate, poly (methyl methacrylate), and polyethylene terephthalate; Silicon; and such Substrates on which aluminum, silver, or a silver/copper/palladium alloy thin film or the like is formed. On these Substrates, other color filter layer, a resin layer, a transistor, a Circuit and the like may be formed. A HMDS-treated Silicon Substrate may also be used.

The formation of each color pixel using a photolithography method can be carried out using a known or conventional device or under known or conventional conditions. For example, the color pixel can be prepared in the following männer. First, a negative resist composition is applied onto a Substrate, and then dried by heat-drying (pre-baking) and/or drying under reduced pressure to remove volatile components such as a solvent from the composition, thereby producing a smooth composition layer. Examples of the application method include a spin coat method, a slit coat method, and a slit-and-spin coat method. The temperature to be employed when heat-drying is carried out is preferably 30 to 120°C, and more preferably 50 to 110°C. The time for the heating is preferably 10 seconds to 60 minutes, and more preferably 30 seconds to 30 minutes. When drying under reduced pressure is carried out, the drying procedure is preferably carried out at a temperature ränge of 20 to 25°C under a pressure of 50 to 150 Pa. The film thickness of the composition layer is not particularly limited, and may be selected appropriately depending on the desired film thickness of the color filter.

Next, the composition layer is exposed to light through a photomask for forming a desired colored pattern. The pattern on the photomask is not particularly limited, and a pattern suitable for the intended application is used. A light source to be used for the exposure to light is preferably a light source capable of generating light having a wavelength of 250 to 450 nm. For example, light having a wavelength of shorter than 350 nm may be cut with a filter capable of cutting light having this wavelength region, or light having a wavelength of around 436 nm, around 408 nm, or around 365 nm may be extracted selectively with a bandpass filter capable of extracting light having those wavelength region. Specific examples include a mercury lamp, a light-emitting diode, a metal halide lamp, and a halogen lamp. A reduced projection light-exposing device or a proximity light-exposing device, such as a mask aligner and a Stepper, is preferably used because the device is capable of emitting a parallel light beam uniformly over the whole area of the exposed surface or accurately aligning the photomask to the Substrate.

A colored pattern is formed on the Substrate by bringing the exposed composition layer into contact with a developing solution to develop the composition layer. By developing, an unexposed area in the composition layer is dissolved in the developing solution and therefore removed. The developing solution is preferably an aqueous solution of an alkaline compound such as potassium hydroxide, sodium hydrogen carbonate, sodium carbonate, or tetramethylammonium hydroxide. The concentration of the alkaline compound in the aqueous solution is preferably 0.01 to 10% by mass, and more preferably 0.03 to 5% by mass. The developing solution may further contain a Surfactant. The developing method may be any of a paddle method, a dipping method, a spray method and the like. Furthermore, during the developing process, the Substrate may be inclined at any angle.

After the developing process, the resultant product is preferably washed with water.

Furthermore, the resultant colored pattern is preferably subjected to post-baking. The temperature for the post-baking is preferably 80 to 250°C, and more preferably 100 to 245°C. The time for the post-baking is preferably 1 to 120 minutes, and more preferably 2 to 30 minutes.

The colored pattern and the colored coating film thus obtained are useful as color filters, and the color filters are useful as color filters used for display devices (for example, a liquid crystal display device and an organic EL device) , electronic paper, solid-state image sensors and the like.

### Examples

Hereinafter, the present invention will be described in more detail with reference to examples, but the present invention is not limited to the following examples. Appropriate modifications can be made to the present invention to the extent that they can be adapted to the gist of the foregoing and following descriptions in the practice of the invention, and all such modifications are covered by the technical scope of the present invention. In the examples, "%" and "part(s)" mean % by mass and part(s) by mass, unless otherwise specified. The structures of the compounds were identified by mass spectrometry (LC; model 1200, manufactured by Agilent, MASS; model LC/MSD, manufactured by Agilent).

### <Synthesis of Dyes>

### [Synthetic Example 1]

50.0 parts of Pink Base manufactured by TAIYO Fine Chemicals Co., Ltd. represented by formula (1), 300 parts of N-methylpyrrolidone (NMP) (manufactured by FUJIFILM Wako Pure Chemical Corporation), 247.0 parts of methyl iodide (manufactured by Tokyo Chemical Industries, Co., Ltd.), and 120.3 parts of potassium carbonate (manufactured by FUJIFILM Wako Pure Chemical Corporation) were mixed at room temperature, and the mixture was heated to 80°C and stirred for 4 hours. After the reaction solution was cooled to room temperature, methyl iodide was distilled off under reduced pressure, and 3300 parts of 1 N hydrochloric acid was placed in the resultant slurry. The resultant precipitate was obtained as residue after suction filtration, and washed with 1000 parts of ion-exchange water and then dried to obtain 50.6 parts of a compound represented by formula (2) . The yield was 97%.

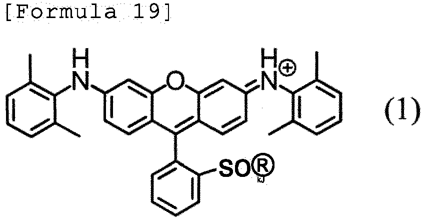

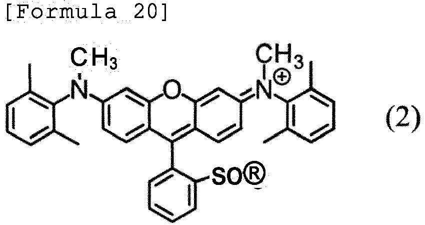

### Identification of Compound Represented by Formula (2)

(Mass spectrometry) ionization mode = ESI⁺: m/z = [M+H]⁺ 603.2

Exact Mass: 602.2

### [Synthetic Example 2]

10.0 parts of a compound, represented by formula (3), 20.0 parts of 2,4,6-trimethylaniline (manufactured by Tokyo Chemical Industries, Co., Ltd.), and 60 parts of NMP (manufactured by FUJIFILM Wako Pure Chemical Corporation) were mixed at room temperature, and the mixture was heated to 160°C and stirred for 7 hours. After the reaction solution was cooled to room temperature, 120 parts of 1 N hydrochloric acid was added. The resultant precipitate was obtained as residue after suction filtration, and washed with 100 parts of ion-exchange water and then dried to obtain 12.4 parts of a compound represented by formula (4) . The yield was 83%.

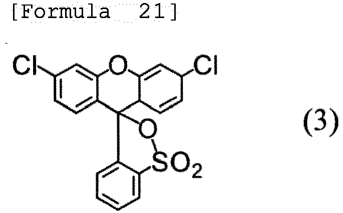

### Identification of Compound Represented by Formula (4)

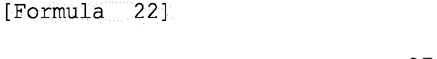

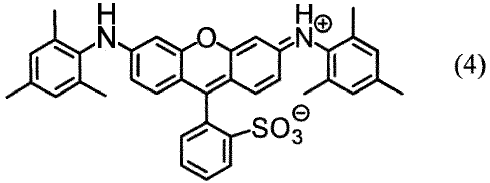

(Mass spectrometry) ionization mode = ESI⁺: m/z = [M+H]⁺ 603.8

Exact Mass: 602.2

### [Synthetic Example 3]

A compound represented by formula (5) was synthesized in the same männer as in Synthetic Example 2, except that 2,4,6-trimethylaniline was replaced with 5-methoxy-2-methylaniline. The yield was 78%.

### Identification of Compound Represented by Formula (5)

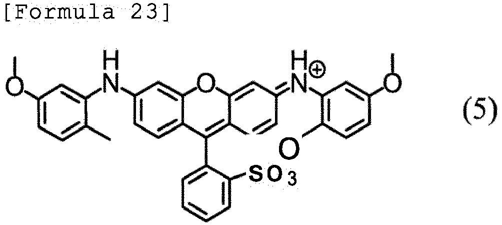

(Mass spectrometry) ionization mode = ESI⁺: m/z = [M+H]⁺ 607.2

Exact Mass: 606.2

### [Synthetic Example 4]

A compound represented by formula (6) was synthesized in the same männer as in Synthetic Example 2, except that 2,4,6-trimethylaniline was replaced with 6-methoxy-2-methylaniline . The yield was 31%.

### Identification of Compound Represented by Formula (6)

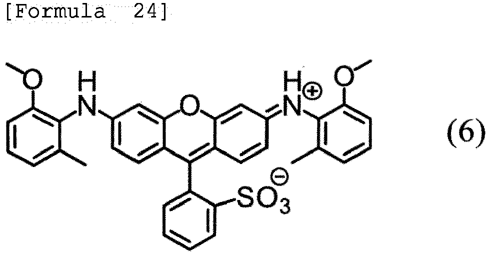

(Mass spectrometry) ionization mode = ESI⁺: m/z = [M+H]⁺ 607.2

Exact Mass: 606.2

### [Synthetic Example 5]

2.0 parts of a compound represented by formula (7), 20.0 parts of N-methylpyrrolidone (NMP) (manufactured by FUJIFILM Wako Pure Chemical Corporation), 16.4 parts of methyl iodide (manufactured by Tokyo Chemical Industries, Co., Ltd.), and 8.0 parts of potassium carbonate (manufactured by FUJIFILM Wako Pure Chemical Corporation) were mixed at room temperature, and the mixture was heated to 80°C and stirred for 16 hours. After the reaction solution was cooled to room temperature, methyl iodide was distilled off under reduced pressure, and 220.0 parts of 1 N hydrochloric acid was placed in the resultant slurry. The resultant precipitate was obtained as residue after suction filtration, and washed with 40 parts of ion-exchange water and then dried to obtain 1.0 part of a compound represented by formula (8) . The yield was 49%.

### Identification of Compound Represented by Formula (7)

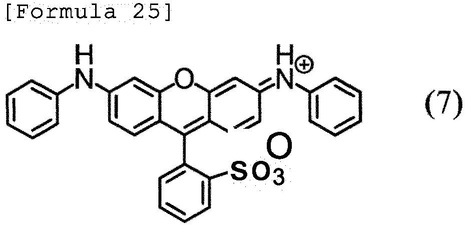

(Mass spectrometry) ionization mode = ESI⁺: m/z = [M+H] ⁺ 519.1

Exact Mass: 518.1

### Identification of Compound Represented by Formula (8)

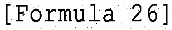

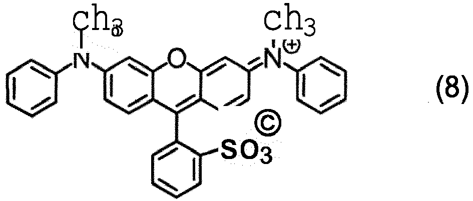

(Mass spectrometry) ionization mode = ESI⁺: m/z = [M+H]⁺ 547.2

Exact Mass: 546.2

### [Synthetic Example 6]

40.6 parts of the compound represented by formula (3) and 8 parts of diethylamine (manuf actured by Tokyo Chemical Industries, Co., Ltd.) were mixed under light-shielding conditions, in the presence of 50 parts of 1-methyl-2-pyrrolidone, and the mixture was stirred at 30°C for 3 hours . After the resultant reaction solution was cooled to room temperature, it was added into a mixture of 400 parts of water and 20 parts of 35% hydrochloric acid, and the mixture was stirred at room temperature for 1 hour to precipitate a crystal. The precipitated crystal was obtained as residue after suction filtration and then dried to obtain 44 parts of a compound represented by formula (I-l-A) .

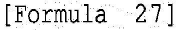

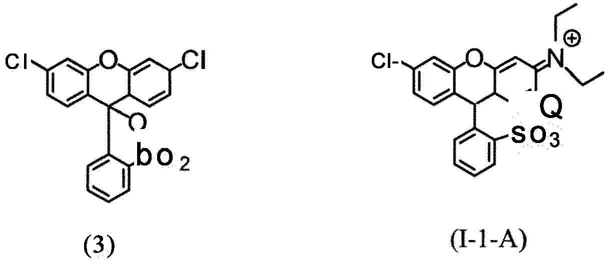

Then, 44 parts of the compound represented by formula (I-l-A) and 21.4 parts of trimethoxy [3-(methylamino)propyl ]silane (manufactured by Tokyo Chemical Industries, Co., Ltd.) were heated at 100°C for 5 hours, in the presence of 50 parts of l-methyl-2-pyrrolidone. After the resultant reaction solution was cooled to room temperature, it was filtered and washed with 100 parts of water, and the resultant crystal was dried to obtain 52 parts of a compound represented by formula (I-1).

### Identification of Compound Represented by Formula (I-D

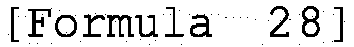

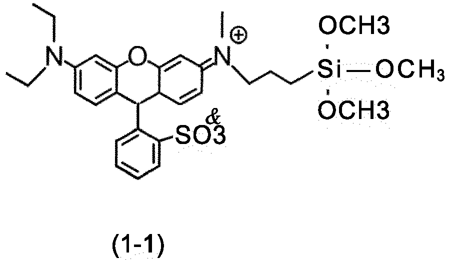

(Mass spectrometry) ionization mode - ESI⁺: m/z = [M+H] ⁺ 599.2

Exact Mass: 598.2

### [Synthetic Example 7]

1 part of Pink Base manufactured by TAIYO Fine Chemicals Co., Ltd. represented by formula (1), 7 parts of N-methylpyrrolidone, 1.0 part of potassium carbonate, and 2.0 parts of ethyl 4-bromobutyrate were added, and the mixture was stirred at 100°C for 7.5 hours . After the mixture was left to cool, 20 parts of 2 N hydrochloric acid was added to the resultant reaction solution, the mixture was extracted with 45 parts of Chloroform twice, and the Chloroform layers were combined and washed with saturated brine solution, and dried over anhydrous magnesium Sulfate. The solvent was distilled off under reduced pressure, and the resultant product was dried at 60°C under reduced pressure to obtain 4.1 parts of a crude compound represented by formula (A-6-IM1) .

### Identification of Compound Represented by Formula (A-6-IM1)

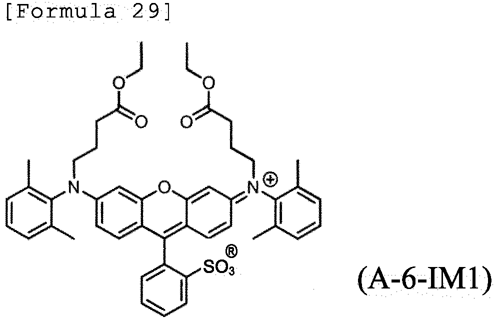

(Mass spectrometry) ionization mode = ESI⁺: m/z = [M+H]⁺ 803.5

Exact Mass: 802.3

To a flask equipped with a condenser tube and a stirrer, 4.1 parts of the compound represented by formula (A-6-IM1), 9.8 parts of methanol, and 3.5 parts of an 8% aqueous solution of sodium hydroxide were added, and the mixture was stirred at room temperature for 6 hours. The resultant reaction solution was added to 30 parts of 2 N hydrochloric acid, and the mixture was stirred at room temperature for 30 minutes to precipitate a crystal . The precipitated crystal was separated by filtration, sufficiently washed with ion-exchange water, and then dried at 60°C under reduced pressure to obtain 1.0 part of a compound (A-6-IM2) .

### Identification of Compound Represented by Formula (A-6-IM2)

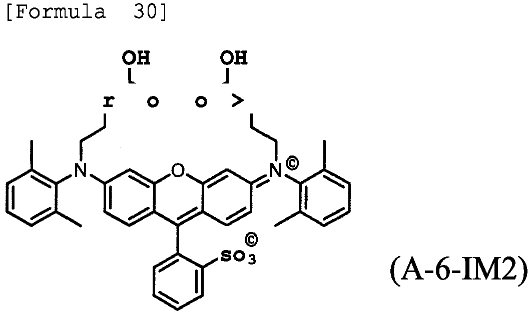

(Mass spectrometry) ionization mode - ESI⁺: m/z = [M+H] ⁺ 747.5

Exact Mass: 746.3

### [Synthetic Example 8]

In a flask equipped with a condenser tube and a stirrer, 15 parts of a dye represented by formula AO-3 (manufactured by TAIYO Fine Chemicals Co., Ltd.), 150 parts of Chloroform, and 8.9 parts of N,N-dimethylf ormamide were placed, and 10.9 parts of thionyl Chloride was then added dropwise to the mixture under stirring while the mixture was maintained at 20°C or lower. After the completion of the dropwise addition, the mixture was heated to 50°C, and the reaction was performed while the mixture was maintained at the same temperature for 5 hours . The reaction solution was then cooled to 20°C. A mixed solution of 12.5 parts of 2-ethylhexylamine and 22.1 parts of triethylamine was added dropwise while the cooled reaction solution was maintained at 20°C or lower under stirring. Then, the reaction was performed while the mixture was stirred at the same temperature for 5 hours. Then, the solvent in the resulting reaction mixture was distilled off using a rotary evaporator, and a small amount of methanol was then added, followed by vigorous stirring. This mixture was added into a mixed solution containing 375 parts of ion-exchange water while being stirred, thereby precipitating a crystal. The precipitated crystal was separated by filtration, sufficiently washed with ion-exchange water, and then dried at 60°C under reduced pressure to obtain 11.3 parts of a dye A3 (mixed dye of dyes A3-1 to A3-8) .

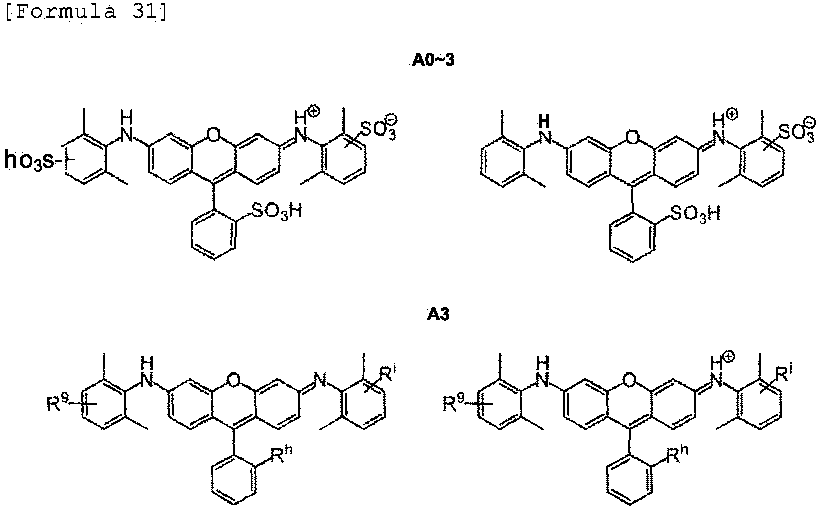

In formula (A3), R9, R^{h} and R¹ each independently represent a hydrogen atom, -S0₃~, -SO3H, or -SÜ2NHRa; and Ra represents 2-ethylhexyl.

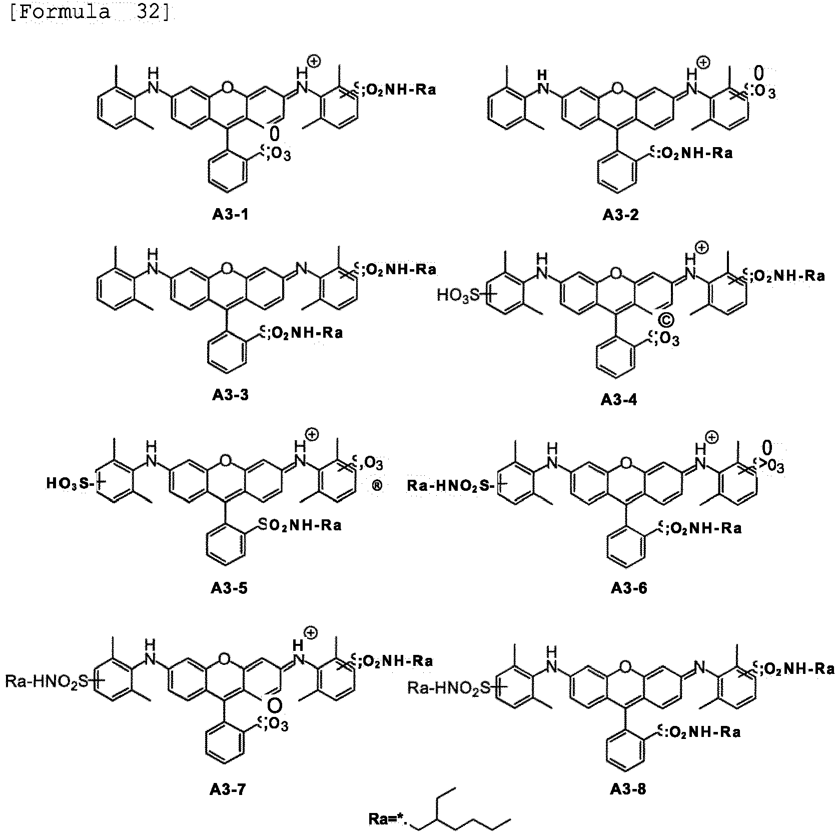

### [Synthetic Example 9]

A compound represented by formula (9) was synthesized in the same männer as in Synthetic Example 2, except that 2,4,6-trimethylaniline was replaced with 0-toluidine. The yield was 90%.

### Identification of Compound Represented by Formula (9)

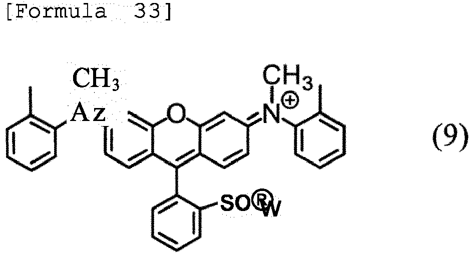

(Mass spectrometry) ionization mode = ESI⁺: m/z = [M+H]⁺ 575.2

Exact Mass: 574.2

### [Synthetic Example 10]

4.00 parts of the compound represented by formula (3), 10.0 parts of 2-aminobiphenyl (manufactured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 9 hours . After the reaction solution was cooled to room temperature, 48.0 parts of 1 N hydrochloric acid was placed. The resultant precipitate was obtained as residue after suction filtration, and further washed with 96.0 parts of 1 N hydrochloric acid. 267 parts of acetone and 267 parts of methanol were added to the resultant residue, the mixture was heated to 50°C and stirred for 1 hour, and the precipitate was obtained as residue after suction filtration. The resultant residue was dried to obtain 4.67 parts of a compound represented by formula (11). The yield was 71%.

### Identification of Compound Represented by Formula (11)

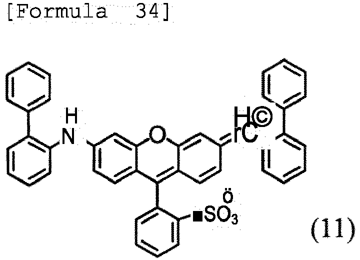

(Mass spectrometry) ionization mode = MALDI-TOF+: m/z = [M+H]⁺ 671.1

Exact Mass: 670.2

### [Synthetic Example 11]

4.00 parts of the compound represented by formula (3), 10.0 parts of 3-aminobiphenyl (manuf actured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 9 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid. 300 parts of acetone and 300 parts of methanol were added to the resultant residue, the mixture was heated to 50°C and stirred for 1 hour, and the precipitate was obtained as residue after suction filtration. The resultant residue was dried to obtain 5.13 parts of a compound represented by formula (12) . The yield was 78%.

### Identification of Compound Represented by Formula (12)

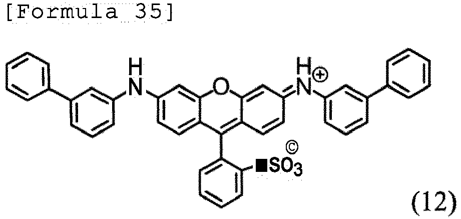

(Mass spectrometry) ionization mode = MALDI-TOF ⁺: m/z = [M+H]⁺ 671.1

Exact Mass: 670.2

### [Synthetic Example 12]

4.00 parts of the compound represented by formula (3), 8.84 parts of 4-tert-butylaniline (manuf actured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 9 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid. 357 parts of acetone and 357 parts of methanol were added to the resultant residue, the mixture was heated to 50°C and stirred for 1 hour, and the precipitate was obtained as residue after suction filtration. The resultant residue was dried to obtain 5.59 parts of a compound represented by formula (13). The yield was 90%.

### Identification of Compound Represented by Formula (13)

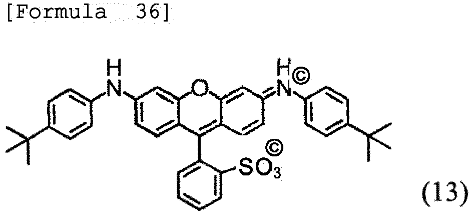

(Mass spectrometry) ionization mode = ESI⁺: m/z = [M+H]⁺ 631.2

Exact Mass: 630.3

### [Synthetic Example 13]

4.00 parts of the compound represented by formula (3), 10.0 parts of 3,5-di-tert-butylaniline (manuf actured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 9 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid. 264 parts of acetone and 264 parts of methanol were added to the resultant residue, the mixture was heated to 50°C and stirred for 1 hour, and the precipitate was obtained as residue after suction filtration. The resultant residue was dried to obtain 4.70 parts of a compound represented by formula (14). The yield was 64%.

### Identification of Compound Represented by Formula (14)

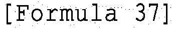

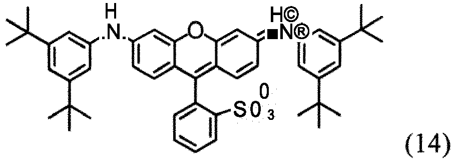

(Mass spectrometry) ionization mode = MALDI-TOF+: m/z = [M+H]⁺ 743.5

Exact Mass: 742.4

### [Synthetic Example 14]

2,6-Diphenylaniline was synthesized with reference to a document (Chun Liu, Xiaoxiao Song, Qijian Ni and Jieshan Qiu; ARKIVOC, 2012,9, 62-75.). Then, 4.00 parts of the compound represented by formula (3), 9.69 parts of 2,6-diphenylaniline, 2.69 parts of zinc Chloride (manufactured by FUJIFILM Wako Pure Chemical Corporation), and 24.0 parts of sulfolane (manufactured by Tokyo Chemical Industries, Co., Ltd.) were mixed at room temperature, and the mixture was heated to 250°C and stirred for 4 hours. After the reaction solution was cooled to room temperature, 48.0 parts of 1 N hydrochloric acid was placed. The resultant precipitate was obtained as residue after suction filtration, and further washed sequentially with 24.0 parts of toluene, 18.0 parts of N,N-dimethylformamide, and 20.0 parts of N, N-dimethylf ormamide . The resultant residue was dried to obtain 5.18 parts of a compound represented by formula (15). The yield was 64%.

### Identification of Compound Represented by Formula (15)

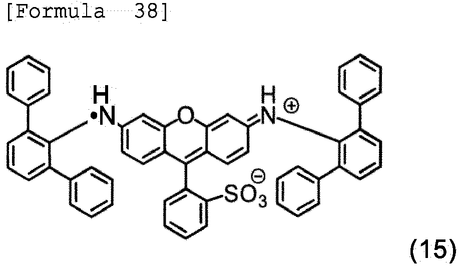

(Mass spectrometry) ionization mode = ESI⁺: m/z = [M+H] ⁺ 823.3

Exact Mass: 822.3

### [Synthetic Example 15]

10.0 parts of the compound represented by formula (3), 16.5 parts of 2,6-dimethylaniline (manufactured by Tokyo Chemical Industries, Co., Ltd.), and 70.0 parts of N-methylpyrrolidone (manufactured by FUJIFILM Wako Pure Chemical Corporation) were mixed at room temperature, and the mixture was heated to 80°C and stirred for 2 hours. After the reaction solution was cooled to room temperature, 140 parts of 1 N hydrochloric acid was placed. The resultant precipitate was obtained as residue after suction filtration, and further washed with 280 parts of 1 N hydrochloric acid. The resultant residue was dried to obtain 9.06 parts of a compound represented by formula (16). The yield was 75%.

### Identification of Compound Represented by Formula (16)

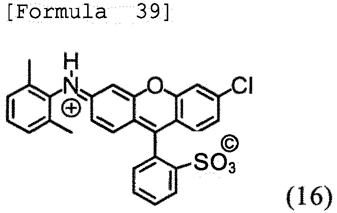

(Mass spectrometry) ionization mode = ESI⁺: m/z = [M+H]⁺ 490.2

Exact Mass: 489.1

4.00 parts of the compound represented by formula (16), 3.80 parts of aniline (manufactured by FUJIFILM Wako Pure Chemical Corporation), and 24.0 parts of N-methylpyrrolidone (manufactured by FUJIFILM Wako Pure Chemical Corporation) were mixed at room temperature, and the mixture was heated to 170°C and stirred for 3 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 36.8 parts of N,N-dimethylformamide . The resultant residue was dried to obtain 3.28 parts of a compound represented by formula (17) . The yield was 74%.

### Identification of Compound Represented by Formula (17)

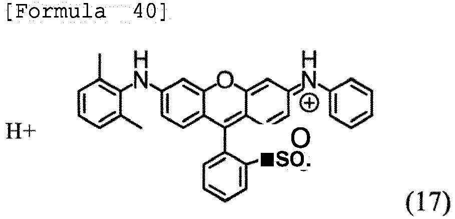

(Mass spectrometry) ionization mode = ESI⁺: m/z = [M+H]⁺ 547.2

### Exact Mass: 546.2

### [Synthetic Example 16]

4.00 parts of the compound represented by formula (16), 6.25 parts of 2,4-dimethoxyaniline (manuf actured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 3 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 42.4 parts of N, N-dimethylformamide . The resultant residue was dried to obtain 2.88 parts of a compound represented by formula (18). The yield was 58%.

### Identification of Compound Represented by Formula (18)

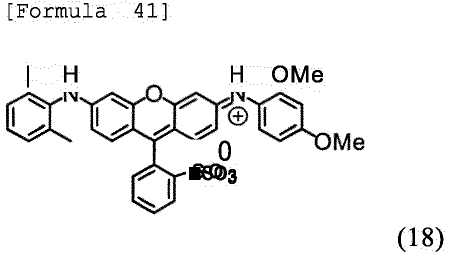

(Mass spectrometry) ionization mode = ESI⁺: m/z = [M+H] ⁺ 607.2

Exact Mass: 606.2

### [Synthetic Example 17]

4.00 parts of the compound represented by formula (16), 5.03 parts of 4-methoxyaniline (manufactured by Tokyo Chemical Industries, Co., Ltd.), and 24.0 parts of N-methylpyrrolidone (manuf actured by FUJIFILM Wako Pure Chemical Corporation) were mixed at room temperature, and the mixture was heated to 170°C and stirred for 3 hours . After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 41.9 parts of N,N-dimethylformamide. The resultant residue was dried to obtain 5.00 parts of a compound represented by formula (19) . The yield was 69%.

### Identification of Compound Represented by Formula (19)

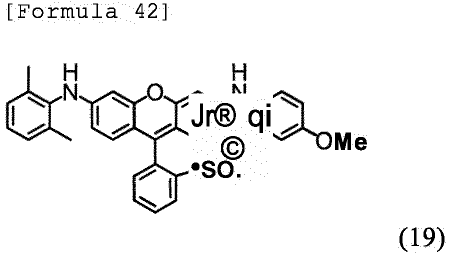

(Mass spectrometry) ionization mode = ESI⁺: m/z = [M+H]⁺ 577.3

Exact Mass: 576.2

### [Synthetic Example 18]

4.00 parts of the compound represented by formula (16), 6.91 parts of 3-aminobiphenyl (manufactured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 5 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 75.2 parts of N,N-dimethylformamide . The resultant residue was dried to obtain 3.79 parts of a compound represented by formula (20). The yield was 75%.

### Identification of Compound Represented by Formula (20)

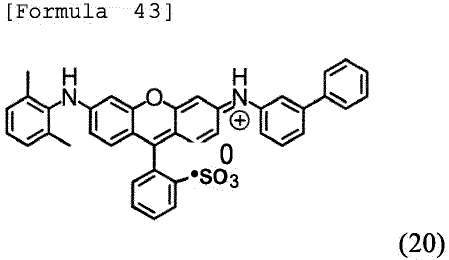

(Mass spectrometry) ionization mode = ESI⁺: m/z = [M+H]⁺ 623.2

Exact Mass: 622.2

### [Synthetic Example 19]

4.00 parts of the compound represented by formula (16), 6.91 parts of 2-aminobiphenyl (manufactured by Tokyo Chemical Industries, Co., Ltd.), and 24.0 parts of N-methylpyrrolidone (manufactured by Tokyo Chemical Industries, Co., Ltd.) were mixed at room temperature, and the mixture was heated to 170°C and stirred for 3 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 44.8 parts of N,N-dimethylf ormamide . The resultant residue was dried to obtain 3.55 parts of a compound represented by formula (21). The yield was 70%.

### Identification of Compound Represented by Formula (21)

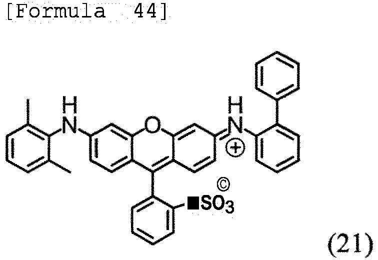

(Mass spectrometry) ionization mode = ESI⁺: m/z = [M+H]⁺ 623.2

Exact Mass: 622.2

### [Synthetic Example 20]

13.0 parts of the compound represented by formula (3), 39.3 parts of 2,6-diphenylaniline, and 78.0 parts of N-methylpyrrolidone (manufactured by FUJIFILM Wako Pure Chemical Corporation) were mixed at room temperature, and the mixture was heated to 85°C and stirred for 5 hours . After the reaction solution was cooled to room temperature, 156 parts of 1 N hydrochloric acid was placed. The resultant precipitate was obtained as residue after suction filtration, and further washed with 312 parts of 1 N hydrochloric acid. The resultant residue was dried to obtain 16.2 parts of a compound represented by formula (22) . The yield was 82%.

### Identification of Compound Represented by Formula (22)

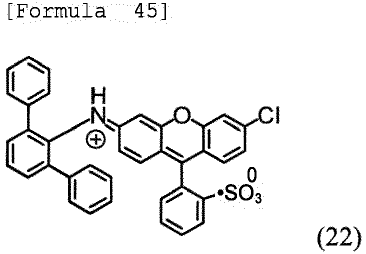

(Mass spectrometry) ionization mode = ESI⁺: m/z = [M+H] ⁺ 614.5

Exact Mass: 613.1

4.00 parts of the compound represented by formula (22), 3.95 parts of 2,6-xylidine (manufactured by FUJIFILM Wako Pure Chemical Corporation), 1.78 parts of zinc Chloride (manufactured by FUJIFILM Wako Pure Chemical Corporation), and 24.0 parts of sulfolane (manufactured by Tokyo Chemical Industries, Co., Ltd.) were mixed at room temperature, and the mixture was heated to 250°C and stirred for 3 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 69.5 parts of N,N-dimethylformamide . The resultant residue was dried to obtain 3.05 parts of a compound represented by formula (23). The yield was 67%.

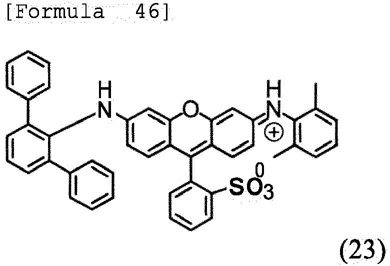

(Mass spectrometry) ionization mode = ESI⁺: m/z = [M+H]⁺ 699.2

Exact Mass: 698.2

### [Synthetic Example 21]

4.00 parts of the compound represented by formula (22), 5.51 parts of 3-aminobiphenyl (manufactured by Tokyo Chemical Industries, Co., Ltd.), and 24.0 parts of N-methylpyrrolidone (manufactured by FUJIFILM Wako Pure Chemical Corporation) were mixed at room temperature, and the mixture was heated to 170°C and stirred for 6 hours. After the reaction solution was cooled to room temperature, 48.0 parts of 1 N hydrochloric acid was placed. The resultant precipitate was obtained as residue after suction Filtration, and further washed with 96.0 parts of 1 N hydrochloric acid and 28.5 parts of N,N-dimethylformamide. The resultant residue was dried to obtain 2.85 parts of a compound represented by formula (24) . The yield was 59%.

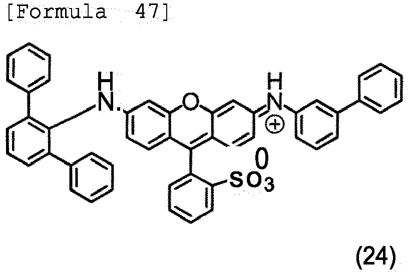

(Mass spectrometry) ionization mode = ESI⁺: m/z = [M+H] ⁺ 747.2

Exact Mass: 746.2

### [Synthetic Example 22]

4.00 parts of the compound represented by formula (22), 5.51 parts of 2-aminobiphenyl (manuf actured by Tokyo Chemical Industries, Co., Ltd.), and 24.0 parts of N-methylpyrrolidone (manuf actured by FUJIFILM Wako Pure Chemical Corporation) were mixed at room temperature, and the mixture was heated to 170°C and stirred for 6 hours. After the reaction solution was cooled to room temperature, 48.0 parts of 1 N hydrochloric acid was placed. The resultant precipitate was obtained as residue after suction filtration, and further washed with 96.0 parts of 1 N hydrochloric acid and 49.2 parts of N,N-dimethylf ormamide. The resultant residue was dried to obtain 2.88 parts of a compound represented by formula (25). The yield was 59%.

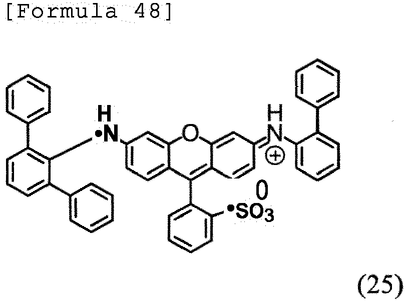

(Mass spectrometry) ionization mode = ESI⁺: m/z = [M+H]⁺ 747.2

Exact Mass: 746.2

### <Synthesis of Resins>

### [Synthetic Example 23: Resin C-l]

In a flask equipped with a stirrer, a dropping funnel, a condenser, a thermometer, and a gas inlet tube, 276.8 g of propylene glycol monomethyl ether acetate was taken, and stirred and heated to 120°C while the flask was being nitrogen purged. Then, a monomer mixture of 92.4 g of 2-ethylhexyl acrylate, 184.9 g of glycidyl methacrylate, and 12.3 g of dicyclopentanyl methacrylate, blended with 35.3 g of t-butylperoxy-2-ethylhexanoate (polymerization Initiator) , was added dropwise from the dropping funnel into the flask over 2 hours . After the completion of the dropwise addition, the copolymerization reaction was performed while the mixture was further stirred at 120°C for 30 minutes to produce an addition copolymer. The inside of the flask was then purged with air. 93.7 g of acrylic acid, 1.5 g of triphenylphosphine (catalyst), and 0.8 g of methoguinone (polymerization inhibitor) were placed in the addition copolymer solution, and the reaction was continued at 110°C over 10 hours, whereby the reaction of an epoxy group derived from glycidyl methacrylate with acrylic acid cleaved the epoxy group and simultaneously, a polymerizable unsaturated bond was introduced into a side chain of the polymer. 24.2 g of succinic anhydride was then added to the reaction System, and the reaction was continued at 110°C over 1 hour, whereby a hydroxy group produced by the cleavage of the epoxy group was reacted with succinic anhydride to introduce a carboxyl group to the side chain to obtain a polymer. Lastly, 383.3 g of propylene glycol monomethyl ether acetate was added to the reaction solution to obtain a polymer (resin (C-l)) solution having a polymer solid content of 40%. The produced Copolymer (polymer; resin (C-l)) had a weight average molecular weight Mw of 6.3 x 10³, and an acid value of 34 mg-KOH/g in terms of solid content.

### [Synthetic Example 24: Resin C-2]

A proper amount of nitrogen was flown into a flask equipped with a reflux condenser, a dropping funnel, and a stirrer to purge the inside of the flask with a nitrogen atmosphere. 280 parts of propylene glycol monomethyl ether acetate was placed in the flask, and then heated to 80°C while being stirred. Then, a solution containing 38 parts of acrylic acid and 289 parts of a mixture (content rate: 1:1) of 3,4-epoxytricyclo [5.2.1.0^{2,6}]decan-8-yl acrylate and 3,4-epoxytricyclo[5.2.1.0^{2,6}]decan-9-yl acrylate dissolved in 125 parts of propylene glycol monomethyl ether acetate was added dropwise thereto using a dropping pump over about 5 hours . Meanwhile, a solution containing 33 parts of 2,2'-azobis (2,4-dimethylvaleronitrile) as a polymerization Initiator dissolved in 235 parts of propylene glycol monomethyl ether acetate was added dropwise into the flask using another dropping pump over about 6 hours . After the completion of the dropwise addition, the resultant solution was held at the same temperature for 4 hours, and then cooled to room temperature to obtain a polymer (resin (C-2)) solution having a polymer solid content of 35.1%. The produced Copolymer (polymer; resin (C-2)) had a weight average molecular weight Mw of 9200, a degree of dispersion of 2.08, and an acid value of 77 mg-KOH/g in terms of solid content. The produced copolymer has the following structural units.

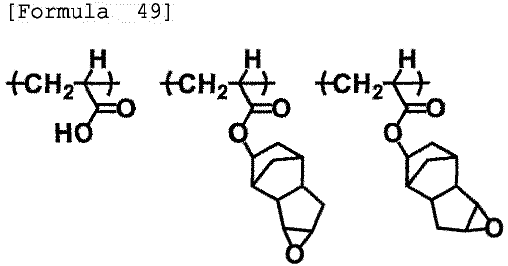

### (Preparation of Dispersion 1)

8 parts of C.I. Pigment Red 122, 3.0 parts of a dispersant (manufactured by BYK, BYKLPN-6919) , 3.0 parts of the resin (C-2) (in terms of solid content), 81 parts of propylene glycol monomethyl ether acetate, and 5 parts of diacetone alcohol were mixed. To the mixture, 300 parts of 0.4-mm zirconia beads were added, followed by shaking for 1 hour using a paint conditioner (manufactured by LAU Corporation) . Then, the zirconia beads were removed by filtration to produce a dispersion 1.

### (Preparation of Dispersion 2)

A dispersion 2 was obtained in the same männer as for the dispersion 1, except that C.I. Pigment Violet 19 was used instead of C.I. Pigment Red 122.

### (Preparation of Dispersion 3)

A dispersion 3 was obtained in the same männer as for the dispersion 1, except that Pink Base manufactured by TAIYO Fine Chemicals Co., Ltd. was used instead of C.I. Pigment Red 122.

### (Preparation of Dispersion 4)

A dispersion 4 was obtained in the same männer as for the dispersion 1, except that the compound obtained in Synthetic Example 1 was used instead of C.I. Pigment Red 122.

### (Preparation of Dispersion 5)

A dispersion 5 was obtained in the same männer as for the dispersion 1, except that the compound obtained in Synthetic Example 2 was used instead of C.I. Pigment Red 122.

### (Preparation of Dispersion 6)

A dispersion 6 was obtained in the same männer as for the dispersion 1, except that the compound obtained in Synthetic Example 3 was used instead of C.I. Pigment Red 122.

### (Preparation of Dispersion 7)

A dispersion 7 was obtained in the same männer as for the dispersion 1, except that the compound obtained in Synthetic Example 4 was used instead of C.I. Pigment Red 122.

### (Preparation of Dispersion 8)

A dispersion 8 was obtained in the same männer as for the dispersion 1, except that the compound obtained in Synthetic Example 5 was used instead of C.I. Pigment Red 122.

### (Preparation of Dispersion 9)

A dispersion 9 was obtained in the same männer as for the dispersion 1, except that the compound obtained in Synthetic Example 9 was used instead of C.I. Pigment Red 122.

### (Preparation of Dispersion 10)

A dispersion 10 was obtained in the same männer as for the dispersion *1,* except that the compound obtained in Synthetic Example 10 was used instead of C.I. Pigment Red 122.

### (Preparation of Dispersion 11)

A dispersion 11 was obtained in the same männer as for the dispersion 1, except that the compound obtained in Synthetic Example 11 was used instead of C.I. Pigment Red 122.

### (Preparation of Dispersion 12)

A dispersion 12 was obtained in the same männer as for the dispersion 1, except that the compound obtained in Synthetic Example 12 was used instead of C.I. Pigment Red 122.

### (Preparation of Dispersion 13)

A dispersion 13 was obtained in the same männer as for the dispersion 1, except that the compound obtained in Synthetic Example 13 was used instead of C.I. Pigment Red 122.

### (Preparation of Dispersion 14)

A dispersion 14 was obtained in the same männer as for the dispersion 1, except that the compound obtained in Synthetic Example 14 was used instead of C.I. Pigment Red 122.

### (Preparation of Dispersion 15)

A dispersion 15 was obtained in the same männer as for the dispersion 1, except that the compound obtained in Synthetic Example 15 was used instead of C.I. Pigment Red 122.

### (Preparation of Dispersion 16)

A dispersion 16 was obtained in the same männer as for the dispersion 1, except that the compound obtained in Synthetic Example 16 was used instead of C.I. Pigment Red 122.

### (Preparation of Dispersion 17)

A dispersion 17 was obtained in the same männer as for the dispersion 1, except that the compound obtained in Synthetic Example 17 was used instead of C.I. Pigment Red 122.

### (Preparation of Dispersion 18)

A dispersion 18 was obtained in the same männer as for the dispersion 1, except that the compound obtained in Synthetic Example 18 was used instead of C.I. Pigment Red 122.

### (Preparation of Dispersion 19)

A dispersion 19 was obtained in the same männer as for the dispersion 1, except that the compound obtained in Synthetic Example 19 was used instead of C.I. Pigment Red 122.

### (Preparation of Dispersion 20)

A dispersion 20 was obtained in the same männer as for the dispersion *1,* except that the compound obtained in Synthetic Example 20 was used instead of C.I. Pigment Red 122.

### (Preparation of Dispersion 21)

A dispersion 21 was obtained in the same männer as for the dispersion 1, except that the compound obtained in Synthetic Example 21 was used instead of C.I. Pigment Red 122.

### (Preparation of Dispersion 22)

A dispersion 22 was obtained in the same männer as for the dispersion 1, except that the compound obtained in Synthetic Example 22 was used instead of C.I. Pigment Red 122.

### [Examples 1 to 27, and Comparative Examples 1 and 2]

The dispersions 1 to 22, the compounds (A-9) to (A-11) of Synthetic Examples 6 to 8, a resin, a polymerizable compound, a polymerization Initiator, a leveling agent, and solvents were mixed to give the final compositions as shown in Tables 3 and 4 below, to obtain negative resist compositions of Examples 1 to 27, and Comparative Examples 1 and 2.

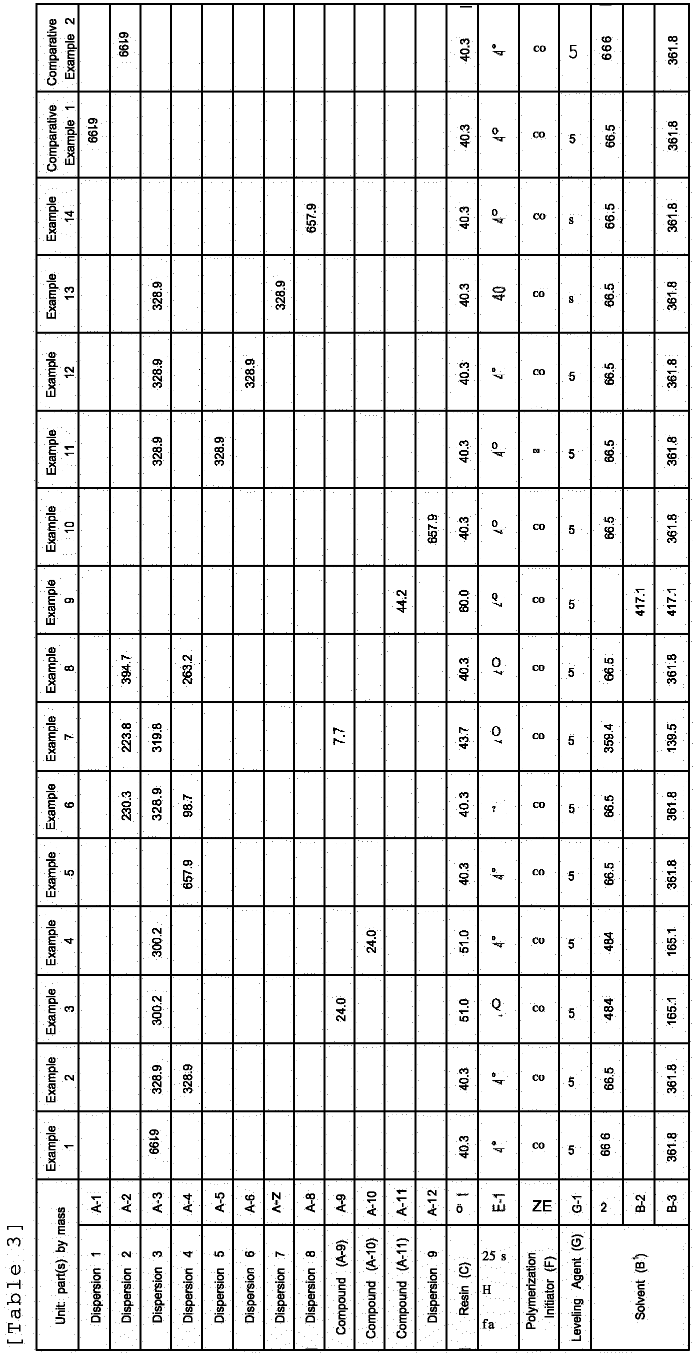

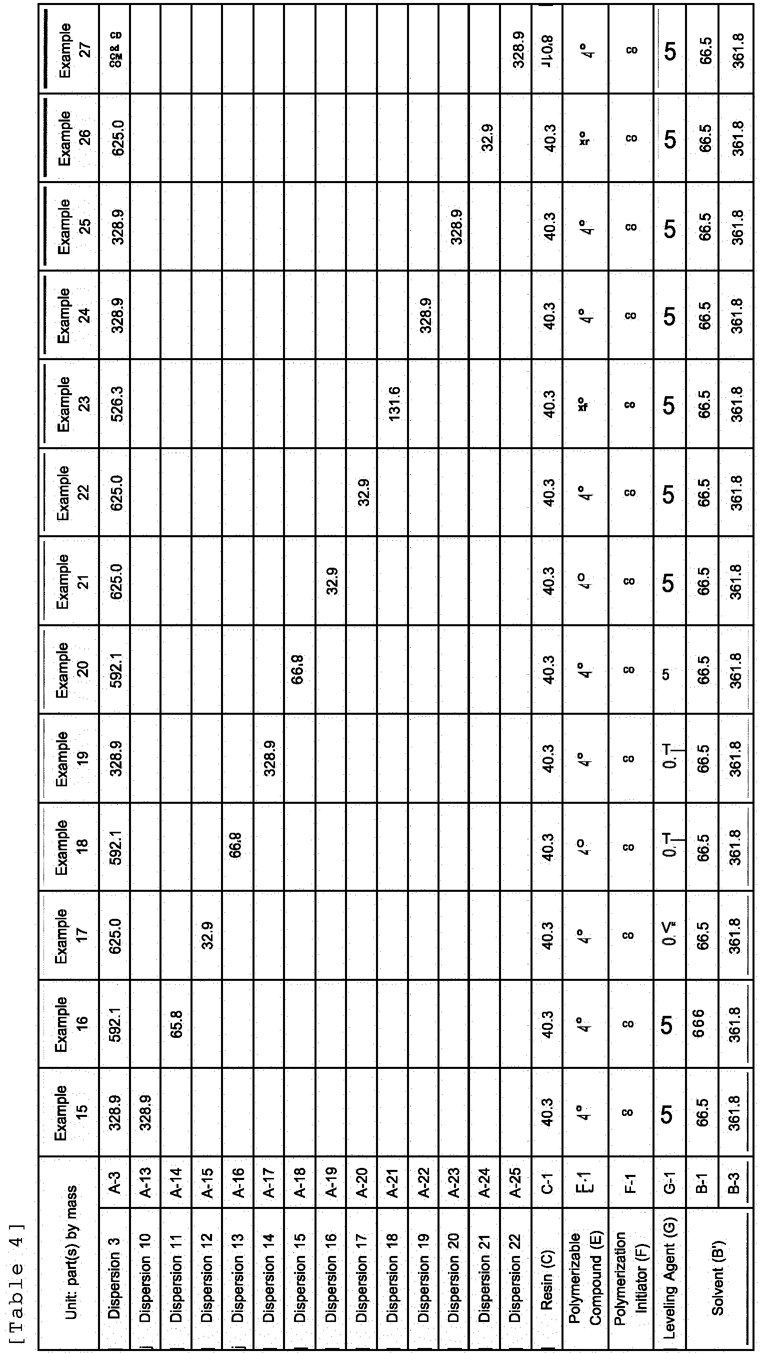

In Tables 3 and 4, the components represent the following compounds :
Compound (A-9) : compound of Synthetic Example 6
Compound (A-10) : compound of Synthetic Example 7
Compound (A-ll) : compound of Synthetic Example 8
Resin (C-l) : resin (C-l) (solid content)
Polymerizable compound (E-l) : dipentaerythritol polyacrylate ("A9550" manufactured by Shin-Nakamura Chemical Co., Ltd.)
Polymerization Initiator (F-l) : N-acetyloxy-1- (4-phenylsulfanylphenyl)-3-cyclohexylpropan-1-one-2-imine (PBG-327; O-acyloxime compound; manufactured by Changzhou Tronly New Electronic Materials Co., Ltd.)
Leveling agent (G-l): polyether-modif ied silicone oil (Toray Silicone SH8400; manufactured by Dow Corning Toray Co., Ltd.)
Solvent (B-l) : diacetone alcohol (DAA)
Solvent (B-2) : ethyl lactate (EL)
Solvent (B-3): propylene glycol monomethyl ether acetate (PGMA)

### <Preparation of Cured Film>

Onto a 5-cm square glass Substrate (Eagle 2000; manufactured by Corning), each negative resist composition was applied by a spin coating method and then pre-baked at 100°C for 3 minutes to form a coloring composition layer. After being left to cool, the coloring composition layer was subjected to light Irradiation in an exposure amount of 60 mJ/cm² (basis: 365 nm) under an air atmosphere using an exposure device (TME-150RSK; manufactured by Topcon Corporation) . Then, the coloring composition layer was subjected to post-baking in an oven at 230°C for 20 minutes to obtain a cured film. In the preparation of the cured film, the film thickness was adjusted so that the transmittance at the maximum absorption wavelength to be described later was 5%.

### <Measurement of Film Thickness>

The film thickness of the obtained cured film on the glass Substrate was measured using a film thickness measuring device (DEKTAK3; manufactured by Japan Vacuum Engineering Co., Ltd.). The results are shown in Tables 5 and 6.

### <Measurement of Maximum Absorption Wavelength and Transmittances>

A spectrum of the obtained cured film on the glass Substrate was measured using a Colorimeter (OSP-SP-200; manufactured by Olympus Corporation) . Specif ically, the maximum absorption wavelength and transmittances at wavelengths of 400 to 700 nm were measured for each cured film. Tables 5 and 6 show the maximum absorption wavelength, the transmittance at the maximum absorption wavelength (T (X max)), the transmittance at a wavelength of 440 nm (T (440 nm)), and the transmittance at a wavelength of 620 nm (T (620 nm)). Tables 5 and 6 also show the ratio (in part(s) by mass) of each colorant contained in the cured film and the colorant weight concentration (in % by mass) contained in the cured film.

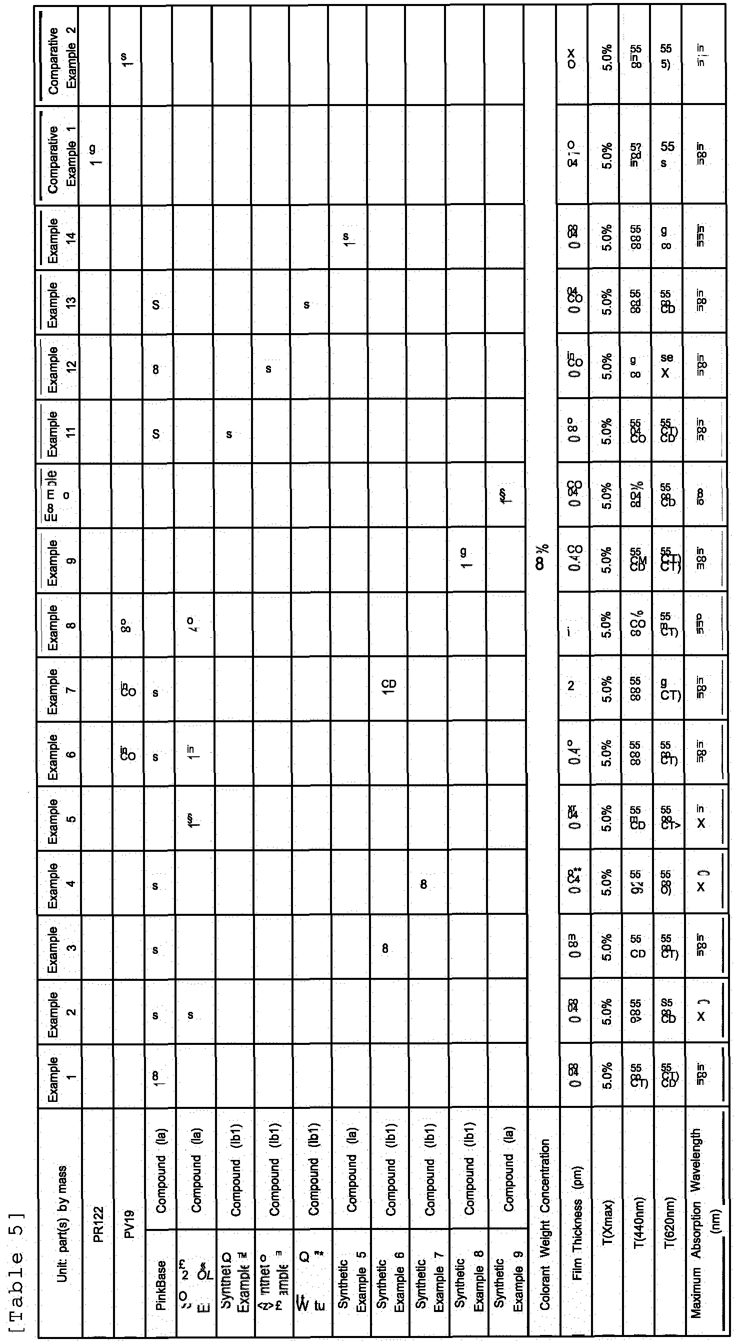

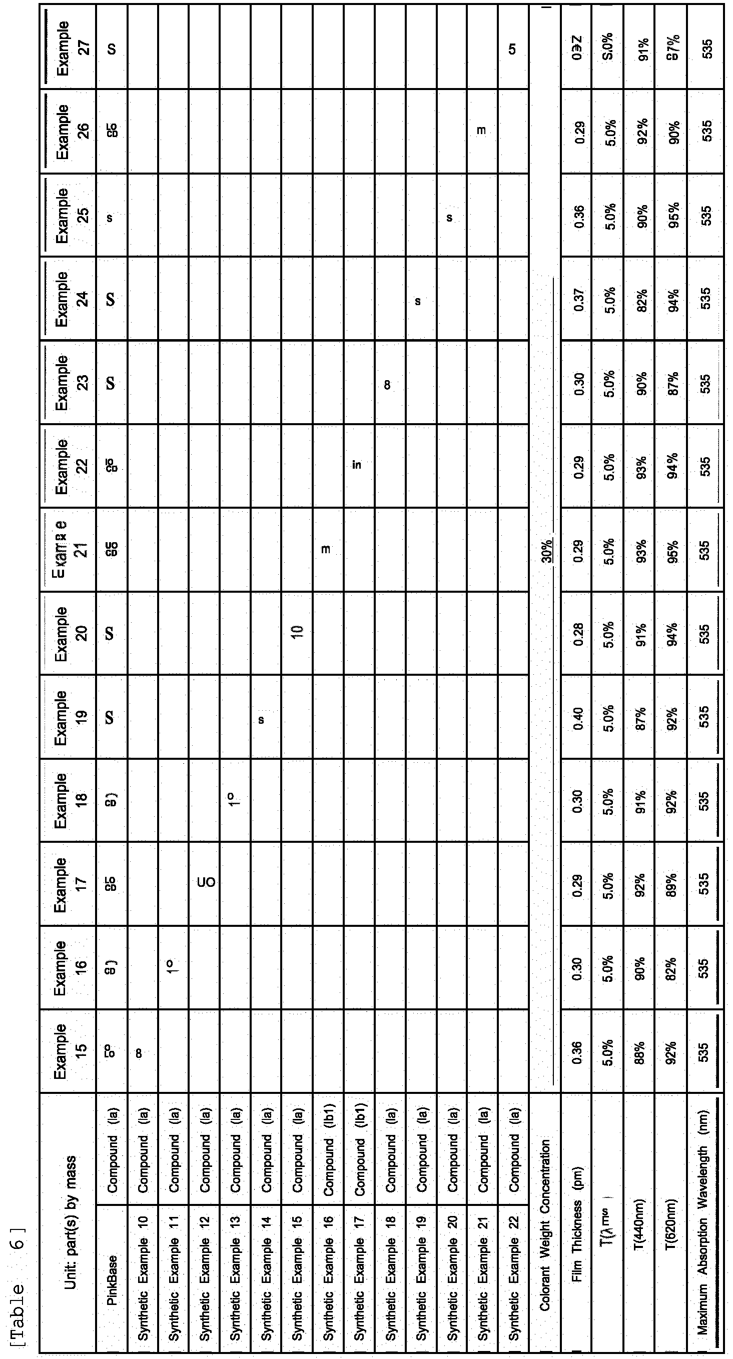

As shown in Tables 5 and 6, the negative resist compositions of Examples 1 to 27 each contained a compound represented by formula (I) above, and, in the cured films obtained from these compositions, the maximum absorption wavelength was present in a wavelength ränge of 500 to 580 nm, and based on the transmittance at the maximum absorption wavelength being 5%, the transmittance at a wavelength of 440 nm was 80% or more and the transmittance at a wavelength of 620 nm was 80% or more. In contrast, the negative resist compositions of Comparative Examples 1 and 2 did not contain a compound represented by formula (I) above, and the cured films obtained from these compositions had a transmittance at a wavelength of 440 nm below 80%.

### Industrial Applicability

A color filter with a high transmittance of light at a wavelength of 440 nm is obtained from the negative resist composition of the present Invention.

## Claims

1. A negative resist composition comprising:
a colorant and a resin,
wherein the colorant contains a xanthene dye, and
a cured film formed from the negative resist composition exhibits a spectrum that satisfies the following condition 1:
[condition 1]
a maximum absorption wavelength is present in a wavelength range of 500 to 580 nm, and based on a transmittance at the maximum absorption wavelength being 5%, a transmittance at a wavelength of 440 nm is 80% or more and a transmittance at a wavelength of 620 nm is 80% or more.

2. The negative resist composition according to claim 1, wherein the transmittance at a wavelength of 620 nm is 90% or more.

3. The negative resist composition according to claim 1 or 2, wherein the xanthene dye is represented by formula (I) : 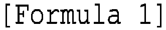 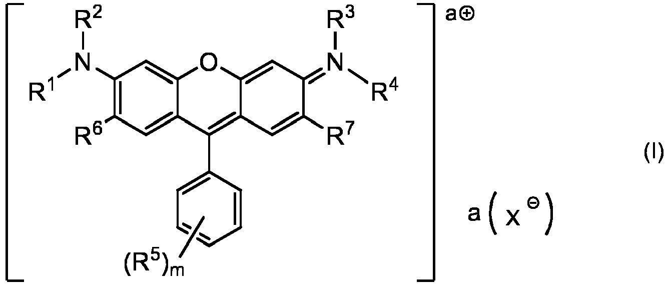
wherein R¹ to R⁴ each independently represent a hydrogen atom, a monovalent saturated hydrocarbon group having 1 to 20 carbon atoms and optionally having a substituent, or a monovalent aromatic hydrocarbon group having 6 to 20 carbon atoms and optionally having a substituent, and -CH₂- contained in the saturated hydrocarbon group is optionally replaced with -O-, -CO-, or -NR¹¹-;
R⁵ represents -OH, -SO₃⁻, -SO₃H, -SO₃⁻Z⁺, -CO₂H, -CO₂⁻Z⁺, -CO₂R⁸, -SO₃R⁸, or -SO₂NR⁹R¹⁰;
R⁶ and R⁷ each independently represent a hydrogen atom or an alkyl group having 1 to 6 carbon atoms;
m represents an integer of 0 to 5; when m is 2 or more, a plurality of R⁵ may be the same or different;
a represents an integer of 0 or 1;
X represents a halogen atom;
Z⁺ represents ⁺N(R¹¹)₄, Na⁺, or K⁺, and four R¹¹ may be the same or different;
R⁸ represents a monovalent saturated hydrocarbon group having 1 to 20 carbon atoms, and a hydrogen atom contained in the saturated hydrocarbon group is optionally replaced with a halogen atom;
R⁹ and R¹⁰ each independently represent a hydrogen atom or a monovalent saturated hydrocarbon group having 1 to 20 carbon atoms and optionally having a substituent, and -CH₂- contained in the saturated hydrocarbon group is optionally replaced with -O-, -CO-, -NH-, or -NR⁸-; and R⁹ and R¹⁰ are optionally bonded to each other to form a 3- to 10-membered heterocycle together with the adjacent nitrogen atom; and
R¹¹ represents a hydrogen atom, a monovalent saturated hydrocarbon group having 1 to 20 carbon atoms, or an aralkyl group having 7 to 10 carbon atoms.

4. The negative resist composition according to claim 3, wherein the xanthene dye is represented by formula (Ia) and/or formula (Ib1): 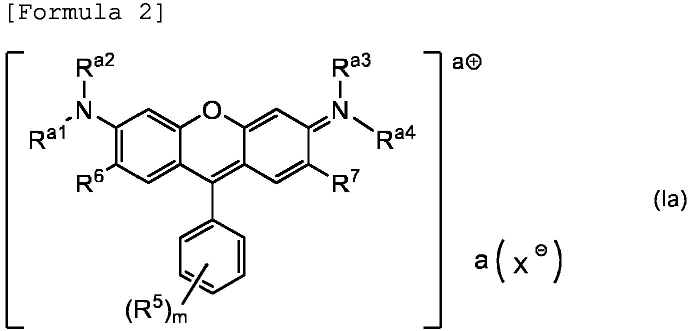 wherein:
R^{a1} and R^{a4} are each independently a monovalent aromatic hydrocarbon group optionally having two or less monovalent saturated aliphatic hydrocarbon groups having 1 to 4 carbon atoms;
R^{a2} and R^{a3} are each independently a hydrogen atom, a methyl group, or an ethyl group; and
R⁵ to R⁷, m, a, and X represent the same meanings as the above; 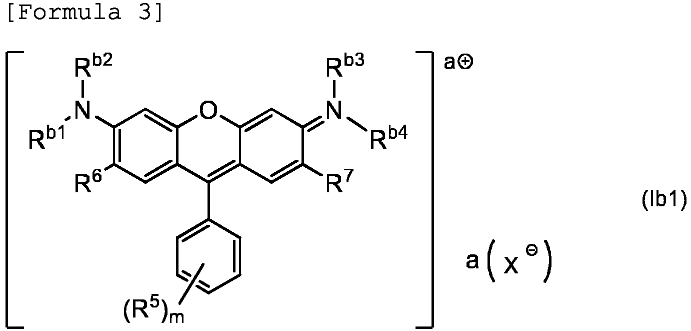
wherein R^{b1} to R^{b4} each independently represent a hydrogen atom, a monovalent saturated hydrocarbon group having 1 to 20 carbon atoms and optionally having a substituent, or a monovalent aromatic hydrocarbon group having 6 to 20 carbon atoms and optionally having a substituent;
at least one saturated hydrocarbon group or aromatic hydrocarbon group contained in R^{b1} to R^{b4} has a halogen atom, -OH, -OR⁸, -CO₂H, -CO₂R⁸, -SO₃⁻, -SO₃H, -SO₃⁻Z⁺, -SR⁸, -SO₂R⁸, -SO₃R⁸, -SO₂NR⁹R¹⁰, or -Si (OR¹²) (OR¹³) (OR¹⁴) as a substituent, or at least one aromatic hydrocarbon group contained in R^{b1} to R^{b4} has three or more monovalent saturated aliphatic hydrocarbon groups having 1 to 4 carbon atoms as substituents;
R¹², R¹³, and R¹⁴ each independently represent a monovalent saturated hydrocarbon group having 1 to 4 carbon atoms, and a hydrogen atom contained in the saturated hydrocarbon group is optionally replaced with a halogen atom; and
R⁵ to R¹⁰, m, a, and X represent the same meanings as the above.

5. The negative resist composition according to claim 4, wherein the xanthene dye is a combination of two or more selected from compounds represented by formula (Ib1), or a combination of a compound represented by formula (Ib1) and a compound represented by formula (Ia).

6. The negative resist composition according to any one of claims 1 to 5, wherein the colorant is a dye alone or a combination of a dye and a red pigment or a purple pigment.

7. The negative resist composition according to any one of claims 1 to 6, further comprising a polymerizable compound and a polymerization initiator.

8. The negative resist composition according to any one of claims 1 to 7, wherein the negative resist composition is capable of forming a cured film with a film thickness of 1.5 µm or less.

9. A color filter formed from the negative resist composition according to any one of claims 1 to 8.

10. A display device comprising the color filter according to claim 9.
